(19) Europäisches Patentamt
European Patent Office
Office européen des brevets

(11) Publication number: **0 242 341 A1**

(12) **EUROPEAN PATENT APPLICATION**

(21) Application number: **87850049.5**

(22) Date of filing: **12.02.87**

(51) Int. Cl.4: **C 07 D 401/12**
C 07 D 403/12,
C 07 D 471/04,
C 07 D 487/04,
A 61 K 31/415, A 61 K 31/44

(30) Priority: **14.02.86 SE 8600658**

(43) Date of publication of application:
**21.10.87 Bulletin 87/43**

(84) Designated Contracting States:
**AT BE CH DE ES FR GB GR IT LI LU NL SE**

(71) Applicant: **Aktiebolaget Hässle**
**Kärragatan 5**
**S-431 83 Mölndal (SE)**

(72) Inventor: **Alminger, Tomas Börje**
**Hassungared PL 42004**
**SE-437 00 Lindome (SE)**

**Brändström, Arne Elof**
**Anders Mattssonsgatan 13B**
**SE-415 06 Göteborg (SE)**

**Carlsson, Stig Åke Ingemar**
**Vallmovägen 3**
**S-435 00 Mölnlycke (SE)**

**Lindberg, Per Lennart**
**Knapehall 64**
**S-436 00 Askim (SE)**

**Nordberg, Peter Mats**
**N. Gubberogatan 12**
**S-416 63 Göteborg (SE)**

**Sunden, Gunnel Elisabeth**
**Kristinehöjdsgatan 15**
**S-412 82 Göteborg (SE)**

(74) Representative: **Hjertman, Ivan T. et al**
**AB ASTRA Patent and Trade Mark Department**
**S-151 85 Södertälje (SE)**

The title of the invention has been amended (Guidelines for Examination in the EPO, A-III, 7.3).

(54) **Benzimidazoles, process for their preparation and preparations containing same.**

(57) Novel compounds of the formula

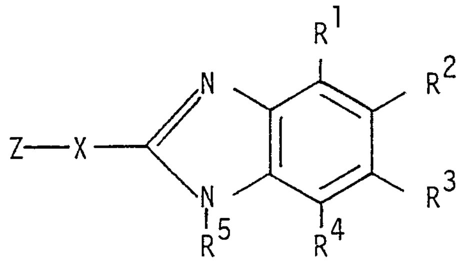

I

pharmaceutical compositions containing such compounds as active ingredient, and the use of the compounds in medicine.

**EP 0 242 341 A1**

**Description**

## Novel Composition of Matter

Field of the invention

The object of the present invention is to provide novel compounds, and therapeutically acceptable salts thereof, which inhibit exogenously or endogenuously stimulated gastric acid secretion and provide cytoprotective effects and thus can be used in the prevention and treatment of peptic ulcer.

The present invention relates to the use of the compounds of the invention or therapeutically acceptable salts thereof, for inhibiting gastric acid secretion as well as providing gastrointestinal cytoprotective effects in mammals and man. In a more general sense, the compounds of the invention may be used for prevention and treatment of gastrointestinal inflammatory diseases in mammals and man, including e.g. gastritis, gastric ulcer, and duodenal ulcer. Furthermore, the compounds may be used for prevention and treatment of other gastrointestinal disorders, where cytoprotective and/or gastric antisecretory effect is desirable e.g. in patients with gastrinomas, in patients with acute upper gastrointestinal bleeding, and in patients with a history of chronic and excessive ethanol consumption. The invention also relates to pharmaceutical compositions containing at least one compound of the invention, or a therapeutically acceptable salt thereof, as active ingredient. In a further aspect, the invention relates to processes for preparation of such new compounds and to novel intermediates in the preparation of the compounds of the invention.

Prior art

Benzimidazole derivatives intended for inhibiting gastric acid secretion are disclosed in the British patent specifications 1 500 043 and 1 525 958, in the US patent 4 182 766, in the European patent specifications 0 005 129, 0 080 602, 0 127 763, 0 134 400, 0 130 729 0 150 586, in the Belgian patent specification 890 024, and in DE 3 415 971. Benzimidazole derivatives proposed for use in the treatment or prevention of special gastrointestinal inflammatory disease are disclosed in the European patent application with publication no. 0 045 200.

The invention

It has been found that the compounds of the formula

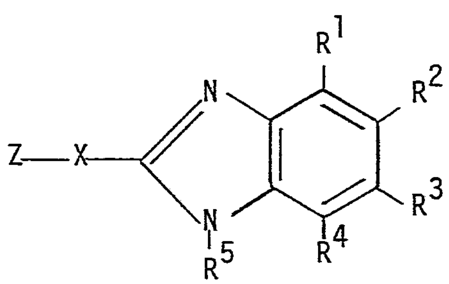

(I)

and therapeutically acceptable salts thereof, in which formula

Z is $Het_1-\underset{R_6}{\underset{|}{CH}}-$ or $Het_2-$;

X is -S- or -SO-;

$Het_1$ is a mono- or bicyclic and $Het_2$ a bicyclic aromatic heterocyclic structure containing 5-10 atoms, optionally substituted by 0-5 substituents, which are the same or different and selected from

a) alkyl containing 1-4 carbon atoms,
b) alkoxy containing 1-3 carbon atoms,
c) amino, mono- or dialkylamino containing 1-3 carbon atoms in the alkyl part(s),
d) alkylthio, containing 1-3 carbon atoms,
e) aryl,
f) arylalkyl containing 1-2 carbon atoms in the alkyl part,
g) aryloxy,
h) arylthio,
i) arylalkoxy containing 1-2 carbon atoms in the alkoxy part or
j) halogen;

The heterocyclic structure Z may contain 1-4 hetero atoms selected from nitrogen, oxygen and sulfur,

however, in all cases with a nitrogen atom separated from the -X- group in formula I by two atoms, one of which, namely that one connected to the -X- group, is a carbon atom.

$R^6$ is H, $CH_3$ or $C_2H_5$;

$R^1$, $R^2$, $R^3$ and $R^4$, which are the same or different, are

a) H,
b) alkyl containing 1-6 carbon atoms,
c) cycloalkyl containing 3-7 carbon atoms,
d) alkoxy containing 1-6 carbon atoms,
e) alkoxyalkyl containing 1-3 carbon atoms in each alkyl part,
f) alkoxyalkoxy containing 1-3 carbon atoms in each alkyl part,
g) halogen,
h) -CN,
i) $-CF_3$,
j) $-NO_2$
k) $COR^7$,
l) alkylthio containing 1-6 carbon atoms in the alkyl part,
m) alkylsulfinyl containing 1-7 carbon atoms in the alkyl part,
n) aryl,
o) arylalkyl containing 1-6 carbon atoms in the alkyl part,
p) aryloxy,
q) arylalkoxy containing 1-6 carbon atoms in the alkyl part or
r) $R^1$ and $R^2$, $R^2$ and $R^3$ or $R^3$ and $R^4$ together with the adjacent carbon atoms in the benzimidazole ring form one or more 5-, 6- or 7-membered rings, which each may be saturated or unsaturated and may contain 0-3 hetero atoms selected from N, S and O and whereby each ring may be optionally substituted with 1-10 substituents selected from alkyl groups with 1-3 carbon atoms, or two or four of the mentioned substituents together form one or two oxo groups (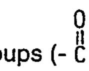), whereby if $R^1$ and $R^2$, $R^2$ and $R^3$ or $R^3$ and $R^4$ together with the adjacent carbon atoms in the benzimidazole ring form two rings, the rings may be condensed with each other;

$R^5$ is

a) H,
b) alkyl containing 1-6 carbon atoms,
c) alkoxyalkyl containing 1-3 carbon atoms in each alkyl part,
d) aryl,
e) arylalkyl containing 1-4 carbon atoms in the alkyl part,
f) aryloxyalkyl containing 1-4 carbon atoms in the alkyl part,
g) alkylthioalkyl containing 1-3 carbon atoms in the alkyl parts,
h) carboxy-substituted alkyl containing 1-6 carbon atoms, optionally in the form of a salt such as the sodium or potassium salt,
i) amino-, monoalkyl (1-3 carbon atoms) -amino-, and dialkyl (1-3 carbon atoms in each alkyl part) -amino- substituted alkyl containing 1-6 carbon atoms, optionally in the form of a salt such as the hydrochloride,
j) cyanoalkyl containing 1-4 carbon atoms in the alkyl part,
k) arylthioalkyl containing 1-4 carbon atoms in the alkyl part,

l) 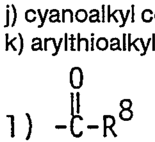 or

m) 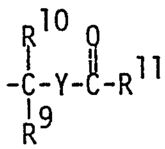

Y is -O- or $-NR^{12}-$;

$R^7$ is

a) alkyl containing 1-6 carbon atoms or
b) alkoxy containing 1-6 carbon atoms;

$R^8$ is

a) alkyl containing 1-6 carbon atoms,
b) arylalkyl containing 1-2 carbon atoms in the alkyl part,
c) aryl,
d) alkoxy containing 1-4 carbon atoms,
e) arylalkoxy containing 1-2 carbon atoms in the alkyl part,

f) aryloxy,
g) amino,
h) mono- or dialkylamino containing 1-4 carbon atoms in the alkyl part(s),
i) arylalkylamino containing 1-2 carbon atoms in the alkyl part or
j) arylamino;

$R^9$ and $R^{10}$ are the same or different and selected from
a) H or
b) alkyl containing 1-4 carbon atoms;

$R^{11}$ is
a) alkyl containing 1-6 carbon atoms,
b) mono- or dihydroxy-substituted alkyl containing 1-6 carbon atoms,
c) amino-, monoalkyl (1-3 carbon atoms) -amino-, and dialkyl (1-3 carbon atoms in each alkyl part) -amino-substituted alkyl containing 1-6 carbon atoms, optionally in the form of a salt such as the hydrochloride,
d) carboxy-substituted alkyl containing 1-7 carbon atoms, optionally in the form of a salt such as the sodium or potassium salt,
e) cycloalkyl containing 3-7 carbon atoms,
f) alkoxy containing 1-6 carbon atoms,
g) mono- or dihydroxysubstituted alkoxy containing 1-6 carbon atoms,
h) amino-, monoalkyl (1-3 carbon atoms) -amino-, and dialkyl (1-3 carbon atoms in each alkyl part) -amino-substituted alkoxy containing 1-6 carbon atoms, optionally in the form of a salt such as the hydrochloride,
i) carboxy-substituted alkoxy containing 1-7 carbon atoms, optionally in the form of a salt such as the sodium or potassium salt,
j) alkoxyalkoxy containing 1-3 carbon atoms in each alkyl part,
k) alkylamino containing 1-6 carbon atoms,
l) dialkylamino containing 1-4 carbon atoms in each alkyl part,
m) aryl, aryloxy or arylalkoxy containing 1-6 carbon atoms in the alkoxy part, the aryl groups are optionally substituted with alkyl containing 1-4 carbon atoms, alkoxy containing 1-4 carbon atoms, halogen, $CF_3$,alkanoyl containing 2-5 carbon atoms, alkoxycarbonyl containing 2-5 carbon atoms or carboxy, whereby the carboxy group optionally may be in the form of a salt such as the sodium salt;

$R^{12}$ is
a) H or
b) alkyl containing 1-4 carbon atoms.

With the provisos that 1) $Het_1$ is neither a pyridine ring nor a pyridine ring condensed with a carbocyclic ring or with an O- or N- containing ring including only one heteroatom, which is attached to the 4-position of the pyridine ring.

2) $Het_1$ is imidazolyl or thiazolyl, unsubstituted or monosubstituted by alkyl or halogen only a) when at least three of $R^1$, $R^2$, $R^3$ and $R^4$ are other than hydrogen, b) when $R^1$, $R^2$, $R^3$ and $R^4$ form at least one ring structure, or c) when $R^5$ is the group $CR^9R^{10}YCOR^{11}$.

3) When $Het_1$ is benzimidazolyl substituted by up to two substituents in the benzene part and unsubstituted or substituted by alkyl, acyl or alkoxycarbonyl on one of the nitrogen atoms, then $R^1$, $R^2$, $R^3$ and $R^4$ are not all hydrogen or when three of $R^1$, $R^2$, $R^3$ and $R^4$ are hydrogen, the fourth is not alkyl or halogen.

4) When $R^5$ is hydrogen and $Het_2$ is a quinoline ring, this ring is substituted in at least one of the positions 5, 6 and 7,

are effective gastric acid secretion inhibitors and also exhibit gastrointestinal cytoprotective effect in mammals and man, and thus fulfil the objectives specified above under "Field of the Invention".

1) A preferred group of compounds of the formula I are compounds wherein the heterocyclic structure Z is

$$Het_1-\underset{\displaystyle R_6}{\underset{|}{CH}}-,$$

2) A preferred group of compounds of the formula I are compounds wherein the heterocyclic structure Z is $Het_2$,

3) A preferred group of compounds of the formula I are compounds wherein the heterocyclic structure Z contains an $sp^2$-hybridized nitrogen atom (i.e. with a free electron pair in the non-bonded $sp^2$-orbital) separated from the -X- group of formula I by two carbon atoms.

4) Particularly preferred among the compounds mentioned in group 3) are compounds with an unsubstituted carbon atom (i.e. bonded to a hydrogen atom) next to the $sp^2$-hybridized nitrogen atom, mentioned above.

5) A preferred group of compounds of the formula I are compounds wherein the heterocyclic structure Z, containing a nitrogen atom separated from the -X- group in formula I by two atoms, has a pKa-value of 2 or more.

6) Particularly preferred among the compounds mentioned in group 5 are compounds with a pKa-value of 3 or more.

7) Particularly preferred among the compounds mentioned in group 5 are compounds with a pKa-value of 4 or more.

8) Further preferred compounds of the formula I are those with the heterocyclic structure Z containing 2-3 hetero atoms and wherein only one of the hetero atoms is selected from oxygen and sulfur, the other hetero atom(s) being nitrogen.

9) A particularly preferred group, are the compounds in which the heterocyclic structure Z contains 2-3 nitrogen atoms and no oxygen or sulfur atoms.

10) Especially preferred compounds of the formula I are those wherein the heterocyclic structure Z contains 2 nitrogen atoms.

11) A preferred group of compounds of the formula I are those, belonging to the mono- or bicyclic aromatic structure $Het_1$, having 2 nitrogen atoms.

12) Especially preferred compounds are those wherein the heterocyclic structure Z is the bicyclic aromatic structure $Het_2$, having 1-2 nitrogen atoms.

13) Additional preferred compounds of the formula I are those, belonging to the bicyclic aromatic structure $Het_2$, containing only one hetero atom, a nitrogen atom.

14) A preferred group of compounds of the formula I are those wherein the heterocyclic structure $Het_1$ or $Het_2$ are substituted by 0-3 substituents, other than hydrogen atoms.

Preferred groups of substituents of the heterocyclic structure $Het_1$ or $Het_2$ are:

a) H,
b) alkyl containing 1-4 carbon atoms,
c) alkoxy containing 1-3 carbon atoms,
d) aryl and
e) arylalkyl

Especially preferred substituents of the heterocyclic structures $Het_1$ or $Het_2$ are H and methyl.

Preferred heterocyclic structures Z are:

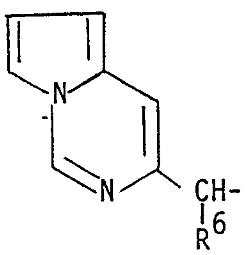
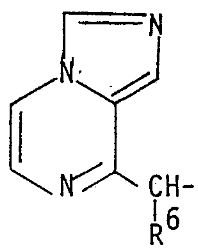
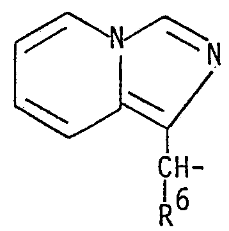
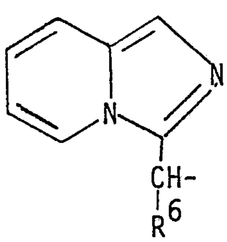
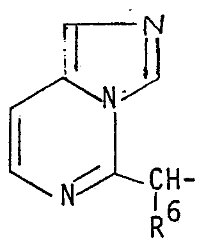
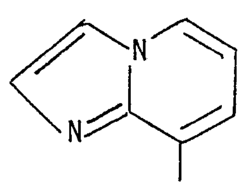
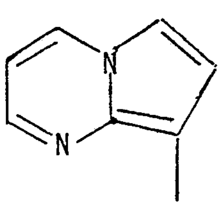
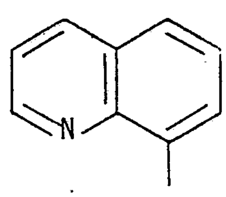
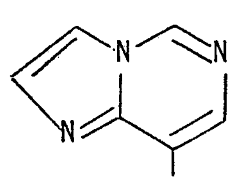

which may be unsubstituted (i.e. substituted by hydrogen atoms) or substituted by 1-3 substituents in the $Het_1$

or $Het_2$ heterocyclic structures.

The preferred group of $R^6$ is H.

A preferred group of X is S.

A preferred group of X is SO.

Preferred groups of the radical $R^5$ are:

a) H,

b) lower alkyl, especially preferred 1-4 carbon atoms,

c) alkoxyalkyl,

d) arylalkyl and

e)

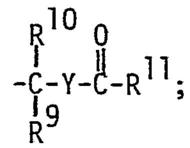

The particularly preferred group of the radical $R^5$ is H.

The preferred group of $R^9$ and $R^{10}$ is H.

The preferred group of Y is -O-.

Preferred groups of the radical $R^{11}$ are:

a) alkyl containing 1-6 carbon atoms, especially methyl,

b) alkoxy containing 1-6 carbon atoms and

c) aryl, especially phenyl;

Preferred groups of compounds of the formula I are compounds wherein $R^1$, $R^2$, $R^3$ and $R^4$ are the same or different and selected from

a) H,

b) alkyl having 1-6 carbon atoms, especially preferred 1-4 carbon atoms, e.g. methyl, ethyl, n-propyl, isopropyl, n-butyl or isobutyl,

c) alkoxy containing 1-6 carbon atoms, especially preferred 1-3 carbon atoms, e.g. methoxy, ethoxy, n-propoxy or isopropoxy

d) or form a saturated or unsaturated 5- or 6-membered ring containing carbon atoms only together with adjacent carbon atoms in the benzimidazole ring

e) or form a saturated or unsaturated 5- or 6-membered ring containing carbon and oxygen atoms together with adjacent carbon atoms in the benzimidazole ring;

Other preferred groups of the radicals $R^1$, $R^2$, $R^3$ and $R^4$ are $-CF_3$ , $-OCF_3$, -phenyl, -phenoxy, -Cl, -CN, $-NO_2$, $-SCH_3$ $-COCH_3$ and $-COOCH_3$;

When $R^5$ is not a hydrogen atom, the preferred benzimidazole structures are those wherein $R^1$ and $R^4$ are the same and $R^2$ and $R^3$ are the same.

Illustrative examples of compounds included in the invention are given in Table 1.

# Table 1

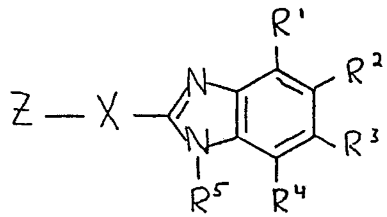

| Z | X | $R^5$ | $R^1$ | $R^2$ | $R^3$ | $R^4$ |
|---|---|---|---|---|---|---|
| 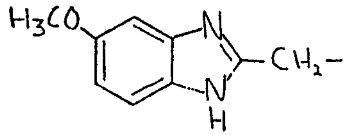 | SO | H | H | $OCH_3$ | H | H |
| 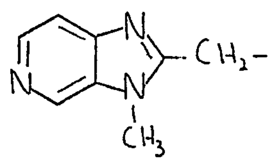 | SO | H | H | $CH_3$ | H | H |
| 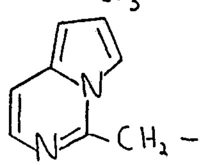 | SO | H | H | $OCH_3$ | H | H |
| 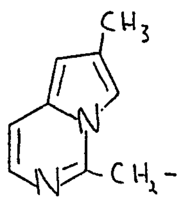 | SO | H | H | $COOCH_3$ | $CH_3$ | H |

cont.

cont. .

| Z | X | $R^5$ | $R^1$ | $R^2$ | $R^3$ | $R^4$ |
|---|---|---|---|---|---|---|
| 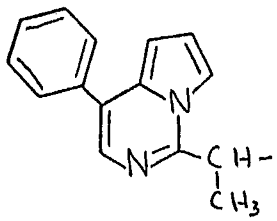 | SO | H | H | $OCH_3$ | H | H |
| 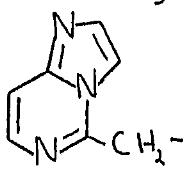 | SO | $CH_3$ | H | $-O-CH_2-O-$ | | H |
| 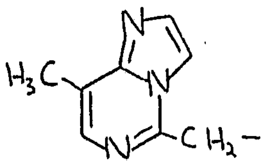 | S | $CH_2CH_3$ | H | H | H | H |
| 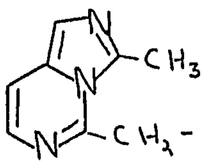 | SO | $CH_2OCOOC_2H_5$ | H | $CH_3$ | $CH_3$ | H |
| 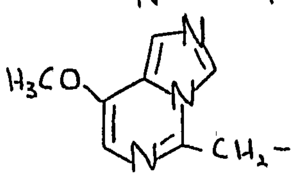 | SO | H | H | $SCH_3$ | H | H |
| 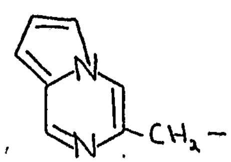 | SO | H | H | $OCH_3$ | H | H |

cont.

cont.

| Z | X | $R^5$ | $R^1$ | $R^2$ | $R^3$ | $R^4$ |
|---|---|---|---|---|---|---|
| 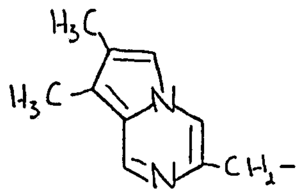 | SO | $CH_3$ | H | H | H | H |
| 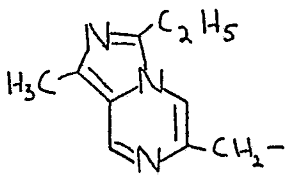 | SO | H | H | $C_2H_5$- | H | H |
| 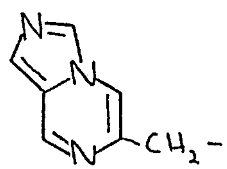 | SO | H | H | $OCH_3$ | H | H |
| 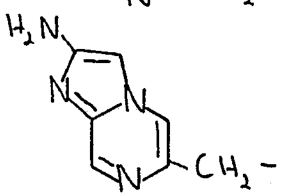 | S | H | H | $OCH_2CH_2$-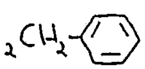 | H | H |
| 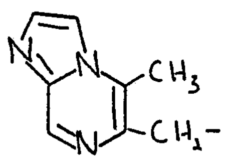 | SO | H | H | $NO_2$ | H | H |
| 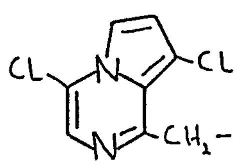 | SO | H | H | $-CH_2CH_2CH_2-$ | | H |

cont.

cont.

| Z | X | $R^5$ | $R^1$ | $R^2$ | $R^3$ | $R^4$ |
|---|---|---|---|---|---|---|
| 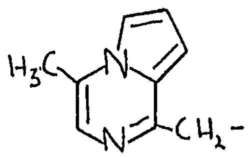 | SO | H | $CH_3$ | H | H | H |
| 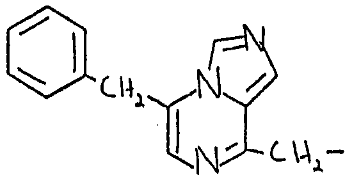 | SO | H | H | $CH_3$ | H | H |
| 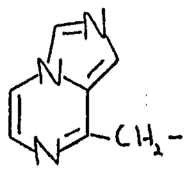 | SO | H | $CH_3$ | $CH_3$ | H | H |
| 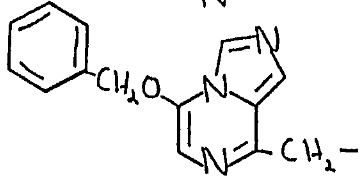 | S | H | H | Cl | H | H |
| 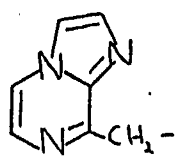 | SO | $COOCH_3$ | H | H | H | H |
| 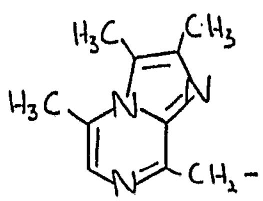 | SO | H | H | $CH_3$ | $CH_3$ | H |

cont.

cont.

| Z | X | $R^5$ | $R^1$ | $R^2$ | $R^3$ | $R^4$ |
|---|---|---|---|---|---|---|
| 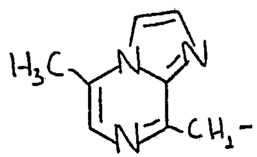 | SO | H | H | $OCH_3$ | H | H |
| 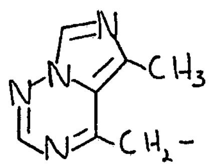 | SO | H | H | $COOCH_3$ | $COOCH_3$ | H |
| 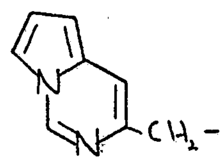 | SO | H | H | $OCH_3$ | H | H |
| 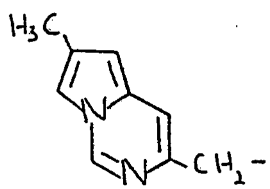 | S | H | $CF_3$ | H | H | H |
| 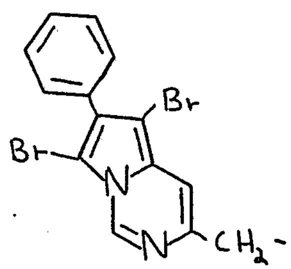 | SO | H | H | Cl | Cl | H |

cont.

cont.

| Z | X | $R^5$ | $R^1$ | $R^2$ | $R^3$ | $R^4$ |
|---|---|---|---|---|---|---|
| 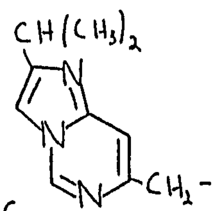 | SO | H | H | 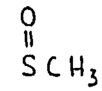 | H | H |
| 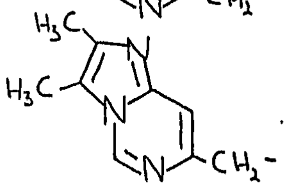 | SO | H | H | 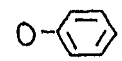 | H | H |
| 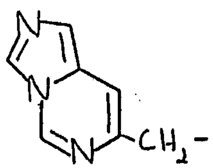 | SO | $CONHCH_3$ | H | $CH_3$ | $CH_3$ | H |
| 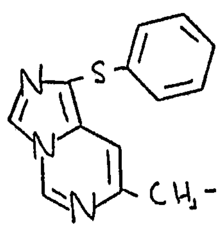 | SO | H | $-CH_2CH_2CH_2CH_2-$ | | H | H |
| 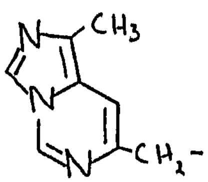 | SO | $CH_3$ | H | H | H | H |

cont.

cont.

| Z | X | $R^5$ | $R^1$ | $R^2$ | $R^3$ | $R^4$ |
|---|---|---|---|---|---|---|
| 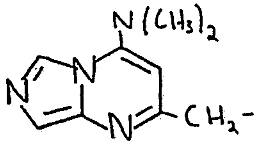 | S | H | H | $C(CH_3)_3$ | H | H |
| 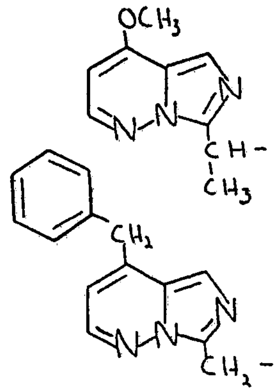 | SO | H | CL | H | H | CL |
| | SO | H | H | $OCH_3$ | H | H |
| 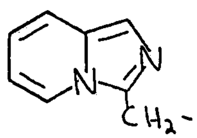 | SO | H | H | $OCH_3$ | H | H |
| 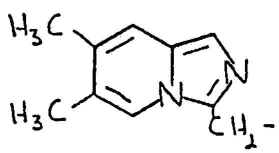 | SO | H | H | $OCH_3$ | H | H |
| 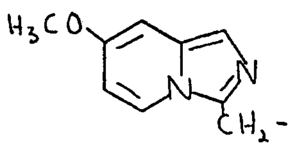 | SO | $CH_2OCOCH_3$ | H | $CH_3$ | $CH_3$ | H |

cont.

cont.

| Z | X | $R^5$ | $R^1$ | $R^2$ | $R^3$ | $R^4$ |
|---|---|---|---|---|---|---|
| 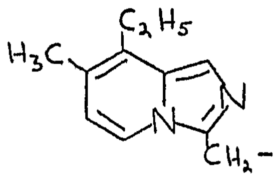 | S | H | H | $SCH_3$ | H | H |
| 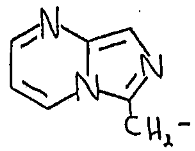 | SO | H | H | $OCH_3$ | H | H |
| 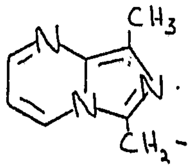 | SO | H | H | 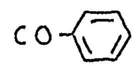 | H | H |
| 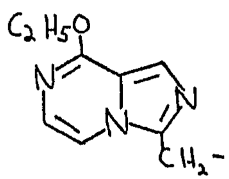 | SO | H | $CH_3$ | $C_2H_5$ | $CH_3$ | H |
| 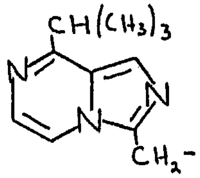 | SO | $CH_2OCOCH_3$ | H | $CH_3$ | $CH_3$ | H |

cont.

cont.

| Z | X | $R^5$ | $R^1$ | $R^2$ | $R^3$ | $R^4$ |
|---|---|---|---|---|---|---|
| 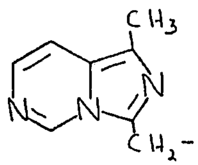 | SO | H | H | CN | H | H |
| 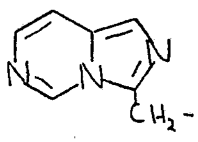 | S | H | H | $NO_2$ | H | H |
| 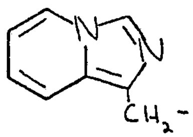 | SO | H | H | $OCH_3$ | H | H |
| 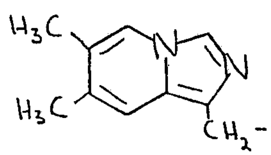 | SO | $CH_2OCO(CH_2)_4COO^-Na^+$ | H | $CH_3$ | $CH_3$ | H |
| 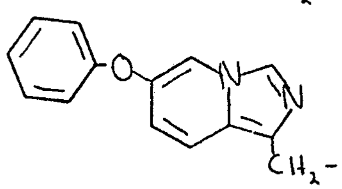 | SO | H | H | $CF_3$ | H | H |
| 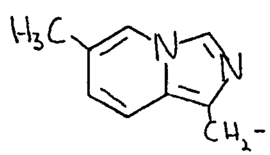 | SO | $CH_2SCH_3$ | H | H | H | H |

cont.

cont.

| Z | X | $R^5$ | $R^1$ | $R^2$ | $R^3$ | $R^4$ |
|---|---|---|---|---|---|---|
| 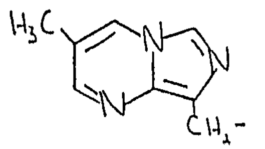 | SO | H | H | $-CH=CH-CH=CH-$ | | H |
| 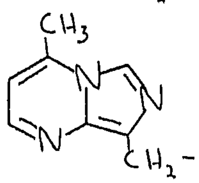 | SO | H | H | $OCH_3$ | H | H |
| 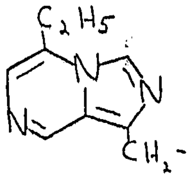 | SO | H | H | $OCH_3$ | H | H |
| 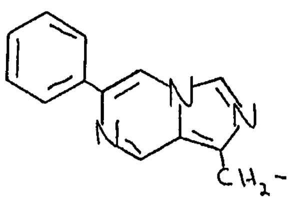 | SO | H | H | $COCH_3$ | H | H |
| 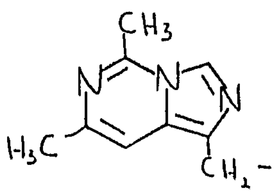 | SO | H | H | 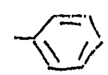 | H | H |

cont.

cont.

| Z | X | $R^5$ | $R^1$ | $R^2$ | $R^3$ | $R^4$ |
|---|---|---|---|---|---|---|
| 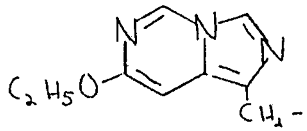 | SO | H | H | $OCH_3$ | H | H |
| 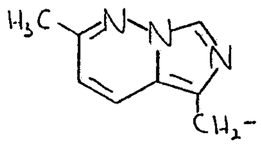 | SO | H | H | $OCH_3$ | H | H |
| 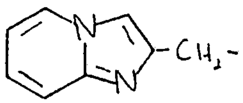 | SO | H | $CH_3$ | H | $CH_3$ | H |
| 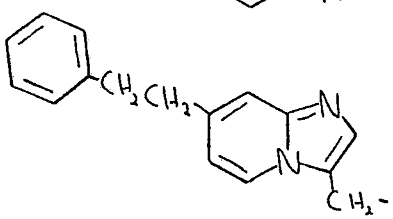 | SO | $CH_2CH_3$ | H | H | H | H |
| 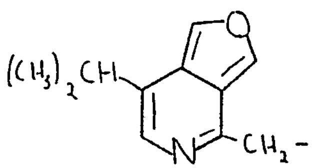 | SO | H | $CH_3$ | $CH_3$ | $CH_3$ | H |
| 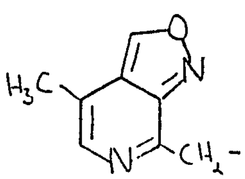 | SO | $CH_2OCO$- 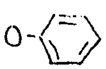 | H | H | H | H |

cont.

cont.

| Z | X | $R^5$ | $R^1$ | $R^2$ | $R^3$ | $R^4$ |
|---|---|---|---|---|---|---|
| 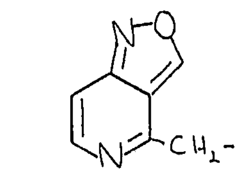 | SO | H | $CH_3$ | H | H | $CH_3$ |
| 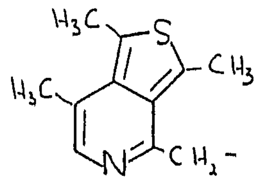 | SO | H | H | $OCH_3$ | H | H |
| 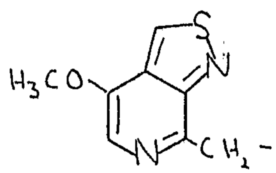 | SO | H | H | $CH_3$ | H | H |
| 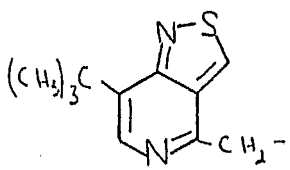 | S | H | H | $CF_3$ | H | H |
| 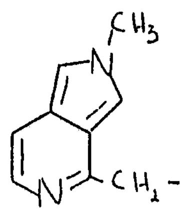 | SO | H | H | $OCH_3$ | H | H |

cont.

cont.

| Z | X | $R^5$ | $R^1$ | $R^2$ | $R^3$ | $R^4$ |
|---|---|---|---|---|---|---|
| 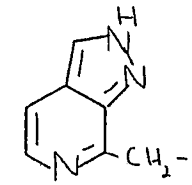 | SO | H | $CH_3$ | $COC_2H_5$ | $CH_3$ | H |
| 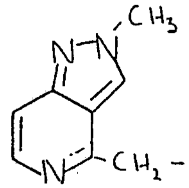 | S | H | H | $CH_2CH_2OCH_3$ | H | H |
| 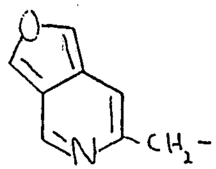 | SO | $CH_2OCOCH_3$ | H | H | H | H |
| 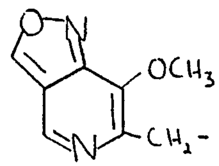 | SO | H | H | $CH(CH_3)_2$ | H | H |
| 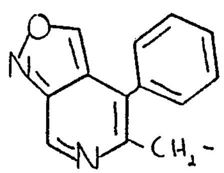 | SO | CONH- 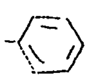 | H | H | H | H |

cont.

cont.

| Z | X | $R^5$ | $R^1$ | $R^2$ | $R^3$ | $R^4$ |
|---|---|---|---|---|---|---|
| 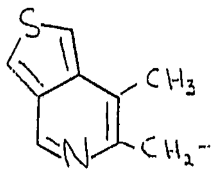 | SO | H | $-CH=CH-CH=N-$ | | H | H |
| 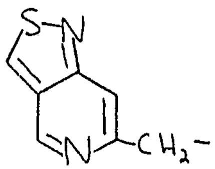 | SO | H | H | $OCH_3$ | H | H |
| 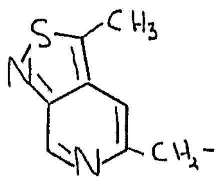 | SO | H | H | $OCH_3$ | H | H |
| 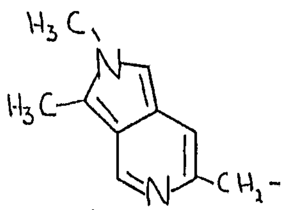 | SO | 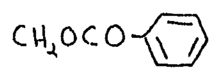 | H | $CH_3$ | $CH_3$ | H |
| 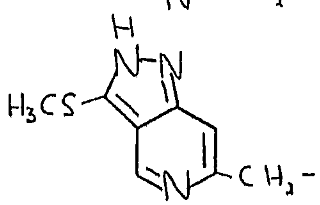 | S | H | H | 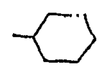 | H | H |

cont.

cont.

| Z | X | $R^5$ | $R^1$ | $R^2$ | $R^3$ | $R^4$ |
|---|---|---|---|---|---|---|
| 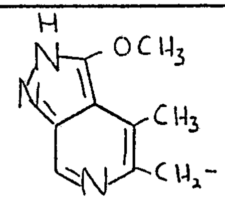 | SO | CO-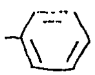 | H | $CH_3$ | $CH_3$ | H |
| 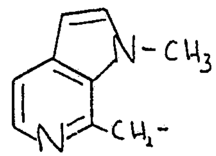 | SO | H | H | $OCH_3$ | H | H |
| 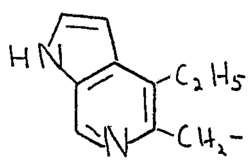 | S | H | $CF_3$ | H | H | H |
| 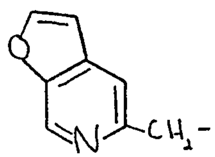 | SO | H | H | $-CH_2CH_2S-$ | | H |
| 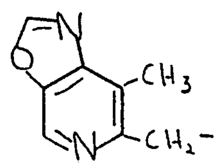 | SO | H | H | $OCH_3$ | H | H |
| 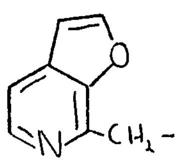 | SO | H | H | $CH_2$-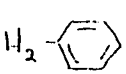 | H | H |

cont.

cont.

| Z | X | $R^5$ | $R'$ | $R^2$ | $R^3$ | $R^4$ |
|---|---|---|---|---|---|---|
| 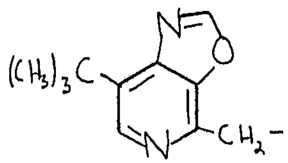 | SO | H | $CH_3$ | $CH_3$ | $CH_3$ | $CH_3$ |
| 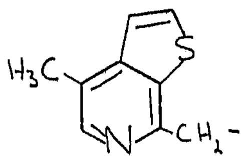 | SO | H | H | $OCH_3$ | H | H |
| 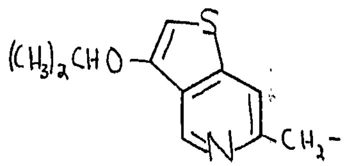 | SO | H | H | $CF_3$ | H | H |
| 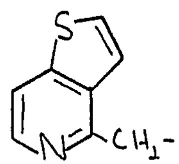 | SO | $CH_2OCOCH_3$ | H | $CH_3$ | $CH_3$ | H |
| 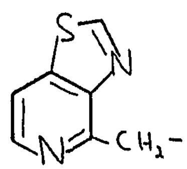 | SO | H | H | $CH_3$ | H | H |
| 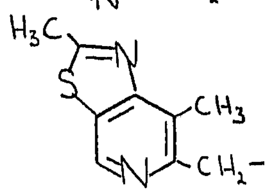 | SO | H | H | $SCH_3$ | H | H |

cont.

cont.

| Z | X | $R^5$ | $R^1$ | $R^2$ | $R^3$ | $R^4$ |
|---|---|---|---|---|---|---|
| 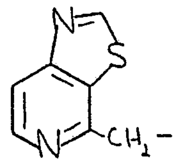 | SO | $\overset{CH_3}{\overset{\mid}{C}H}OCOOC_2H_5$ | H | H | H | H |
| 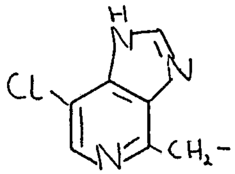 | S | H | H | $COOCH_3$ | H | H |
| 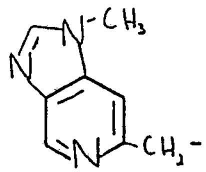 | SO | H | H | $CH_3$ | $CH_3$ | H |
| 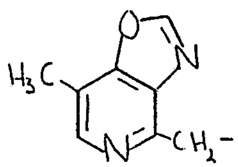 | SO | H | H | $OCH_3$ | H | H |
| 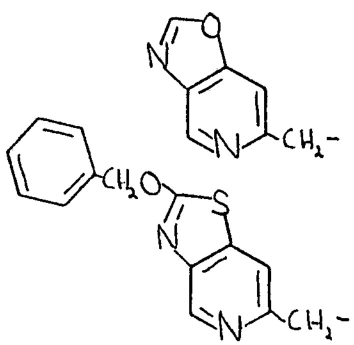 | SO | H | H | $OCH_3$ | H | H |
|  | SO | H | H | $COCH_3$ | H | H |

cont.

cont.

| Z | X | $R^5$ | $R^1$ | $R^2$ | $R^3$ | $R^4$ |
|---|---|---|---|---|---|---|
| 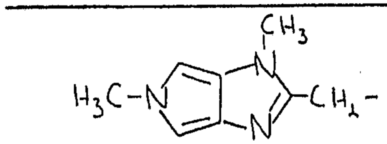 | S | H | H | CL | H | H |
| 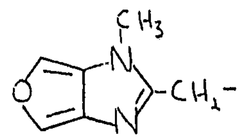 | SO | H | H | $CH_3$ | $CH_3$ | H |
| 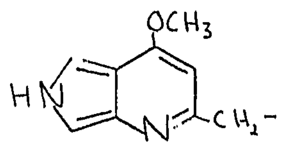 | SO | $CH_2OCO-$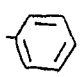 | H | H | H | H |
| 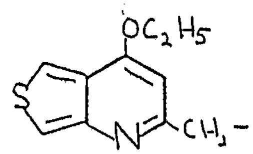 | SO | H | H | $OCH_3$ | H | H |
| 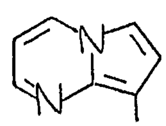 | SO | H | H | $OCH_3$ | H | H |
| 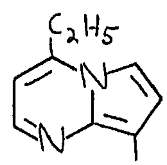 | S | H | $CH_3$ | H | H | H |

cont.

cont.

| Z | X | $R^5$ | $R^1$ | $R^2$ | $R^3$ | $R^4$ |
|---|---|---|---|---|---|---|
| 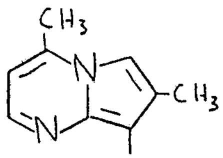 | SO | $CH_2CH_2OCH_3$ | H | H | H | H |
| 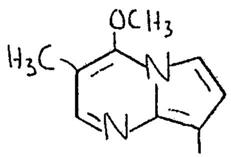 | SO | $CH_2CH_3$ | H | $CH_3$ | $CH_3$ | H |
| 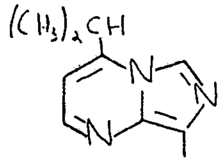 | SO | H | H | Cl | H | H |
| 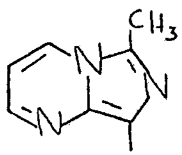 | SO | H | H | 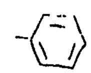 | H | H |
| 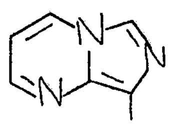 | S | H | H | $CH(CH_3)_2$ | H | H |
| 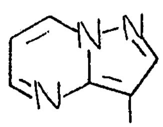 | SO | H | H | $C_2H_5$ | H | H |

cont.

cont.

| Z | X | $R^5$ | $R^1$ | $R^2$ | $R^3$ | $R^4$ |
|---|---|---|---|---|---|---|
| 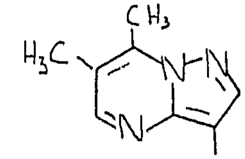 | SO | H | H | O-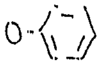 | H | H |
| 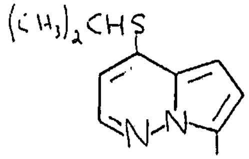 | SO | H | H | $COCH_3$ | H | H |
| 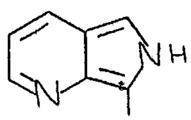 | SO | H | $-CH=CH-CH=N-$ | | H | H |
| 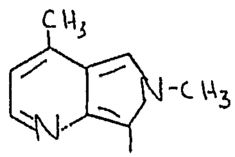 | SO | H | H | $OCH_3$ | H | H |
| 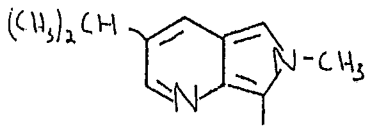 | SO | H | H | $-\overset{\overset{O}{\|}}{S}-CH_3$ | H | H |
| 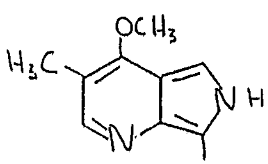 | SO | $CH_2OCO$-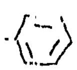 | H | H | H | H |

cont.

cont.

| Z | X | $R^5$ | $R^1$ | $R^2$ | $R^3$ | $R^4$ |
|---|---|---|---|---|---|---|
| 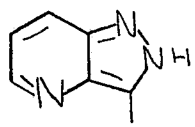 | SO | H | H | $OCH_3$ | H | H |
| 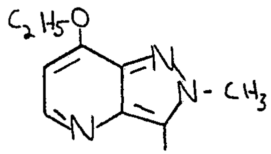 | SO | H | H | $COOCH_3$ | H | H |
| 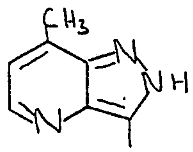 | SO | H | H | 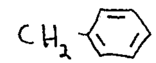 | H | H |
| 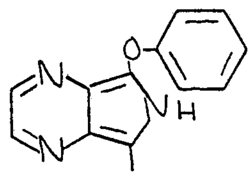 | S | H | H | $CF_3$ | H | H |
| 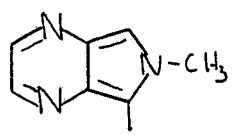 | SO | H | H | $CH(CH_3)_2$ | H | H |
| 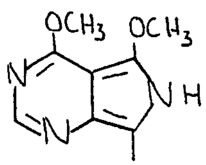 | SO | $(CH_2)_4COO^- Na^+$ | H | H | H | H |

cont.

cont.

| Z | X | $R^5$ | $R^1$ | $R^2$ | $R^3$ | $R^4$ |
|---|---|---|---|---|---|---|
| 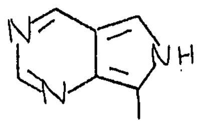 | SO | H | H | $OCH_3$ | H | H |
| 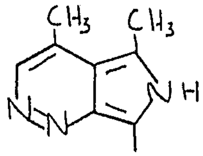 | SO | H | H | $SCH_3$ | H | H |
| 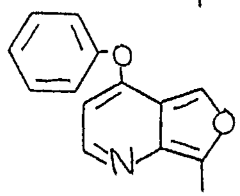 | S | $CH_3$ | H | H | H | H |
| 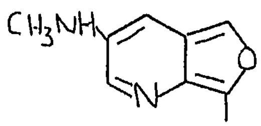 | S | H | H | $NO_2$ | H | H |
| 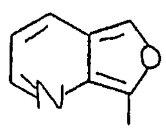 | SO | H | H | 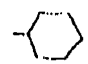 | H | H |
| 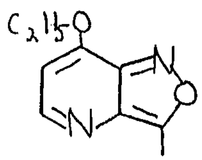 | SO | $CH_2CH_2$- 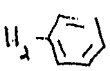 | H | H | H | H |

cont.

cont.

| Z | X | $R^5$ | $R^1$ | $R^2$ | $R^3$ | $R^4$ |
|---|---|---|---|---|---|---|
| 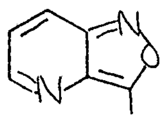 | SO | $CH_2CH_3$ | H | H | H | H |
| 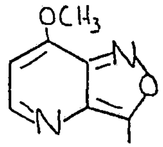 | SO | H | H | 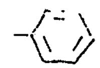 | H | H |
| 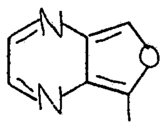 | SO | $CH_2OCOCH_3$ | H | $CH_3$ | $CH_3$ | H |
| 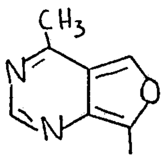 | S | H | H | $OC_2H_5$ | H | H |
| 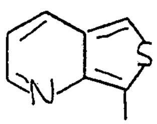 | SO | H | H | $CF_3$ | H | H |
| 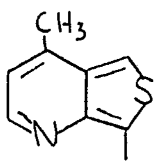 | SO | H | H | $OCH_3$ | H | H |

cont.

cont.

| Z | X | $R^5$ | R' | $R^2$ | $R^3$ | $R^4$ |
|---|---|---|---|---|---|---|
| 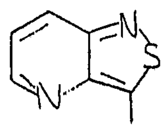 | SO | $CH_3$ | H | H | H | H |
| 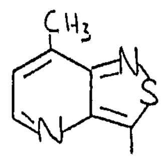 | SO | H | H | $COOCH_3$ | H | H |
| 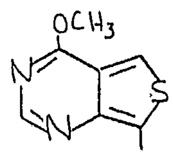 | SO | H | H | $CH_3$ | $CH_3$ | H |
| 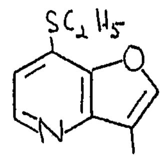 | SO | H | H | $OCF_3$ | H | H |
| 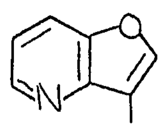 | S | H | H | $OCH_3$ | H | H |
| 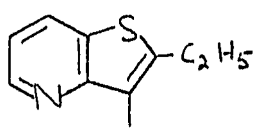 | SO | H | H | CN | H | H |

cont.

cont.

| Z | X | $R^5$ | $R'$ | $R^2$ | $R^3$ | $R^4$ |
|---|---|---|---|---|---|---|
| 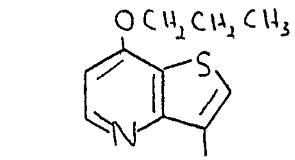 | SO | H | H | $-CH=CH-CH=CH-$ | | H |
| 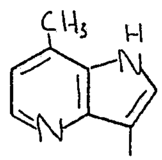 | SO | H | H | $OCH_3$ | H | H |
| 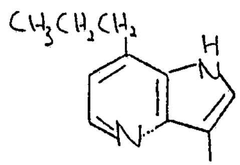 | S | H | H | $NO_2$ | H | H |
| 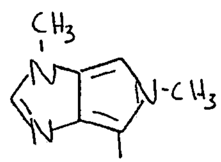 | SO | H | $CH_3$ | $C_2H_5$ | $CH_3$ | H |
| 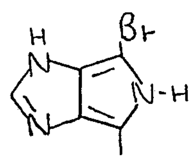 | S | H | H | $-CH_2CH_2CH_2-$ | | H |
| 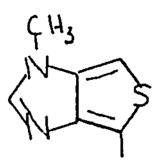 | SO | H | H | $CF_3$ | H | H |

cont.

cont.

| Z | X | $R^5$ | $R^1$ | $R^2$ | $R^3$ | $R^4$ |
|---|---|---|---|---|---|---|
| 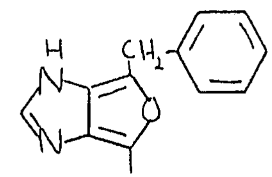 | SO | H | H | $OC_2H_5$ | H | H |
| 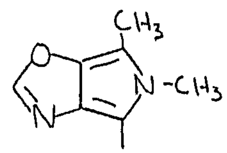 | SO | $COOCH_3$ | H | H | H | H |
| 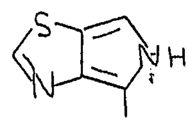 | SO | H | H | $CH_3$ | $CH_3$ | H |
| 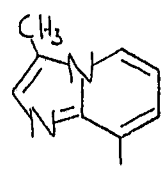 | SO | H | $CH_3$ | $CH_3$ | $CH_3$ | H |
| 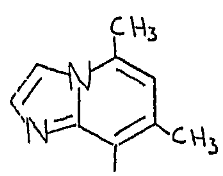 | S | H | H | $-OCH_2O-$ | | H |
| 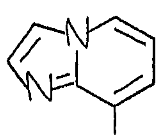 | SO | H | H | $OCH_3$ | H | H |

cont.

cont.

| Z | X | $R^5$ | $R^1$ | $R^2$ | $R^3$ | $R^4$ |
|---|---|---|---|---|---|---|
| 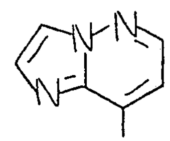 | SO | H | H | $-CH_2CH_2S-$ | | H |
| 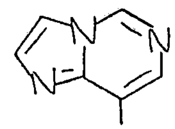 | SO | H | H | $OCH_3$ | H | H |
| 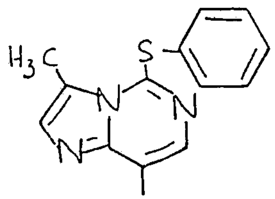 | SO | H | $CH_3$ | H | $CH_3$ | $CH_3$ |
| 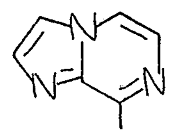 | SO | H | H | $OCH_3$ | H | H |
| 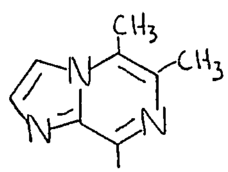 | S | H | H | $NO_2$ | H | H |
| 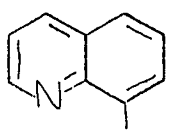 | SO | $CH_3$ | H | H | H | H |

cont.

cont.

| Z | X | $R^5$ | $R^1$ | $R^2$ | $R^3$ | $R^4$ |
|---|---|---|---|---|---|---|
| 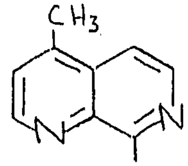 | SO | H | H | $CF_3$ | H | H |
| 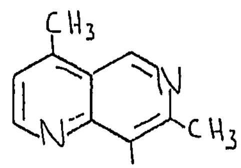 | SO | H | $CH_3$ | $CH_3$ | $CH_3$ | $CH_3$ |
| 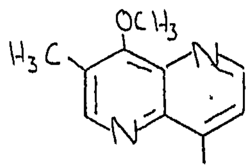 | SO | $CH_3$ | H | $CH_3$ | $CH_3$ | H |
| 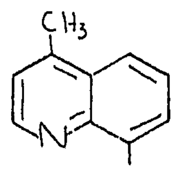 | SO | $CH_3$ | H | H | H | H |
| 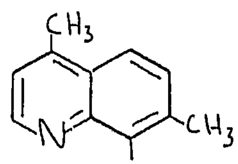 | S | H | H | H | H | H |
| 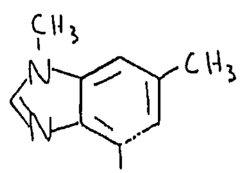 | SO | $CH_2CH_3$ | H | H | H | H |

cont.

cont.

| Z | X | $R^5$ | $R^1$ | $R^2$ | $R^3$ | $R^4$ |
|---|---|---|---|---|---|---|
| 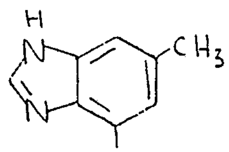 | S | H | H | $OCH_2CH_2OCH_3$ | H | H |
| 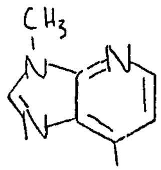 | SO | H | H | $OCH_3$ | H | H |
| 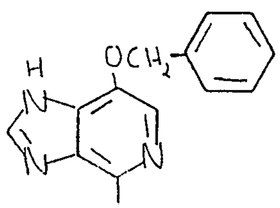 | SO | H | $CH_3$ | H | $CH_3$ | H |

Preparation

The compounds of formula I above may be prepared according to the following methods:

a) Oxidizing a compound of the formula I,

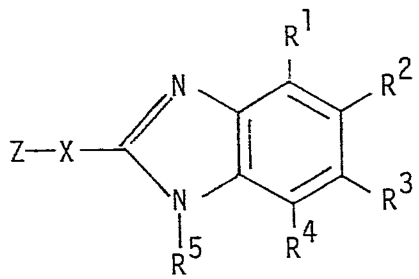

I

wherein X is S and Z, $R^1$, $R^2$, $R^3$, $R^4$ and $R^5$ have the meanings given, to give a compound of the same formula I wherein X is SO.

This oxidation may be carried out by using an oxidizing agent selected from the group consisting of nitric acid, hydrogen peroxide, peracids, peresters, dinitrogentetraoxide, iodosobenzene, N-halosuccinimide, 1-chlorobenzotriazole, t-butylhypochlorite, diazabicylco-[2,2,2]-octane bromine complex, sodium metaperiodate, selenium dioxide, manganese dioxide, chromic acid, cericammonium nitrate, bromine, chlorine and sulfuryl chloride.

The oxidation may also be carried out enzymatically by using an oxidating enzyme or microbiotically by using a suitable microorganism.

b) Reacting a compound of the formula II,

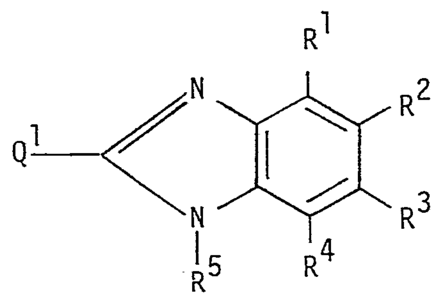

II

with a compound of the formula III or IV,

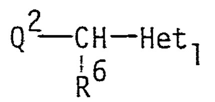

III

$Q^2—Het_2$

IV

in which $R^1$, $R^2$, $R^3$, $R^4$, $R^5$, $R^6$, $Het_1$ and $Het_2$ are as defined previously and wherein one of $Q^1$ and $Q^2$ is SH and the other is a leaving group, gives a compound of the formula I wherein X is S.

Examples of leaving groups $Q^1$ and $Q^2$ in the compounds II, III and IV are halogens, preferably chlorine, bromine or iodine, acyloxy radicals, for example residues of strong organic sulfonic acids, for instance of an arylsulfonic acid, for example tosyloxy or an alkylsulfonic acid, for example mesyloxy, alkylmercapto groups, for example methylmercapto, alkylsulfinyl groups, for example methylsulfinyl and the like.

Thus $Q^1$ and $Q^2$ when designating leaving groups may be a reactive esterified hydroxy group. The esterification may be carried out with an organic acid or with an inorganic acid such as HCl, HBr or $H_2SO_4$.

The reaction of a compound of formula II above with a compound of formula III or IV is conveniently carried out in the presence of a suitable solvent that is inert under the reaction conditions utilized as described hereinafter. The reaction may further be carried out in the presence of a suitable base. Suitable bases include, for example, inorganic bases such as sodium or potassium hydroxide, sodium or potassium alkoxide, sodium or potassium hydride and the like, organic bases such as tertiary amines, for example triethylamine and the like.

The reaction may also be carried out in the presence of an organic acid or with an inorganic acid such as HCl, HBr or $H_2SO_4$.

Suitable solvents for the above described reaction include, for example, alcohols, preferably lower alkanols

such as methanol and ethanol, mixtures of such alcohols with water, ethers, such as tetrahydrofuran, halogenated hydrocarbons, such as methylene chloride. Aprotic solvents such as ethers and halogenated carbons are necessary in the case of sodium and potassium hydride.

The reaction of the compounds of formulas II and III or II and IV may be carried out at a temperature between the ambient temperature and the boiling temperature of the reaction mixture.

c) Reacting a compound of the formula V,

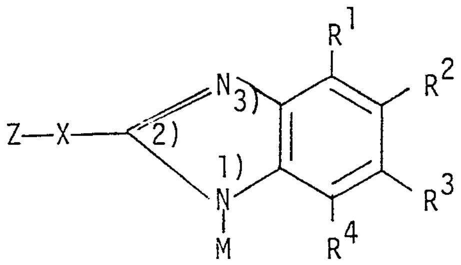

V

wherein $R^1$, $R^2$, $R^3$, $R^4$, X and Z are as defined under formula I and M is either a metal cation such as Na+, K+ and Li+ or a quaternary ammonium ion, such as tetrabutylammonium, with a compound of the formula VI

$R^5Q^3$ VI

wherein $R^5$ is as defined under formula I and $Q^3$ is halogen such as Cl, Br or I, or a functionally equivalent group.

Suitable solvents for the reaction of a compound of formula V with a compound of formula VI are hydrocarbons such as toluene and benzene and halogenated hydrocarbons such as methylene chloride and chloroform.

The reaction may also be carried out in a twophase system with water as one component and a hydrocarbon or a halogenated hydrocarbon as the other.

The reaction of the compound of formula V and VI may be carried out at a temperature between the ambient temperature and the boiling temperature of the reaction mixture.

Normally, if the benzimidazole moiety is asymmetrically substituted, both the N(1) and the N(3) derivatives are obtained and therefore, if necessary, the two components have to be separated. This may be done by recrystallizations or by extractive or chromatographic techniques.

If desired, the compound of the formula I thus obtained, when X is -S-, is converted to a physiologically acceptable salt or oxidized to form a compound of the formula I wherein X is -SO-.

Depending on the process conditions and the starting materials, the end products of the formula I wherein X is S is obtained either as the free base or as a salt. The end products of the formula I wherein X is -SO-are obtained as the free base. Both the free base and the salts of these end products are included within the scope of the invention. Thus, basic, neutral or mixed salts may be obtained as well as hemi, mono, sesqui or polyhydrates. Acid addition salts of the new sulfides may in a manner known per se be transformed into free base using basic agents such as alkali or by ion exchange. The free bases of the sulfides obtained may also form salts with organic or inorganic acids. In the preparation of acid addition salts preferably such acids are used which form suitable therapeutically acceptable salts.

Examples of such acids are hydrohalogen acids, sulfonic acid, phosphoric acid, nitric acid and perchloric acid; aliphatic, alicyclic, aromatic or heterocyclic carboxyl or sulfonic acids, such as formic acid, acetic acid, propionic acid, succinic acid, glycolic acid, lactic acid, malic acid, tartaric acid, citric acid, ascorbic acid, maleic acid, hydroxymaleic acid, pyruvic acid, phenylacetic acid, benzoic acid, p-aminobenzoic acid, p-hydroxybenzoic acid, salicylic acid or p-aminosalicylic acid, embonic acid, methanesulfonic acid, ethanesulfonic acid, hydroxyethanesulfonic acid, ethylenesulfonic acid, halogenbenzenesulfonic acid, toluenesulfonic acid, naphtylsulfonic acid or sulfanilic acids, methionine, tryptophane, lysine or arginine.

These or other salts of the new sulfide compounds, as e.g. picrates, may serve as purifying agents of the free bases obtained. Salts of the bases may be formed, separated from solution and then the free base can be recovered in higher purity from a new salt solution.

Also included in the scope of the invention are alkaline salts of the compounds of the formula I wherein X is SO and $R^5$ is H. These alkaline salts are exemplified by the formula VII

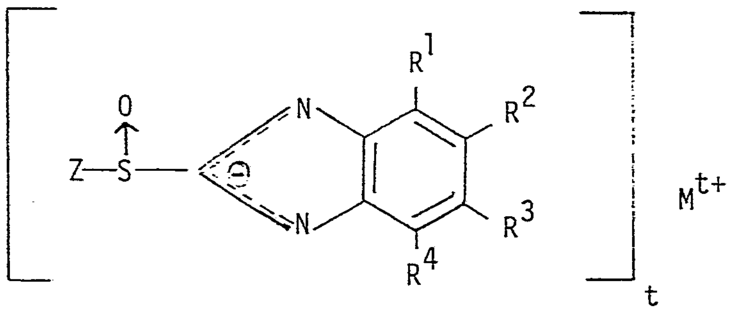

VII

wherein t is 1, 2 or 4; $M^{t+}$ is $Li^+$, $Na^+$, $K^+$, $Mg^{2+}$, $Ca^{2+}$, $Ti^{4+}$, $N^+(R)_4$ or

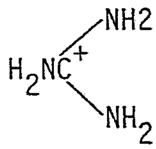

, wherein R is an alkyl group containing 1-4 carbon atoms.

Particularly preferred are the $Na^+$, $Ca^{2+}$ and $Mg^{2+}$ salts. The salts of the formula VII exhibit improved stability which means that they can be stored for extended periods of time without substantial degradation.

The alkaline salts of the invention of the formula VII are prepared following the method according to EP O 124 495, that is by reacting a compound of the formula I wherein X is SO and $R^5$ is H with a base capable of releasing the cation

$M^{t+}$

wherein $M^{t+}$ is as defined above, whereafter the salt of the formula VII obtained is isolated.

Examples of bases capable of releasing the cation $M^{t+}$, and examples of reaction conditions are given below.

a) Salts of the formula VII wherein M is Li, Na or K are prepared using LiOH, NaOH or KOH in an aqueous or nonaqueous medium or with LiOR, $LiNH_2$, $LiNR_2$, NaOR, $NaNH_2$, $NaNR_2$ KOR, $KNH_2$ or $KNR_2$, wherein R is an alkyl group containing 1-4 carbon atoms, in a nonaqueous solvent such as an alcohol (only for the alcoholates),e.g. ROH, or in an ether such as tetrahydrofuran.

Racemates obtained can be separated according to known methods, e.g. recrystallization from an optically active solvent, use of microorganisms, reactions with optically active acids forming diastereomeric salts which can be separated, (e.g. separation based on different solubilities of the diastereomers), acylation of the benzimidazole nitrogen ($R^5$=H) or another nitrogen or oxygen atom in a substituent by an optically active activated carboxylic acid (e.g. acid chloride), followed by chromatographic separation and deacylation.

Suitable optically active acids for salt formation are the L- and D-forms of tartaric acid, di-o-tolyl-tartaric acid, malic acid, mandelic acid, camphorsulfonic acid or quinic acid and for acylation O-methylmandelic acid. Preferably the more active part of the two antipodes is isolated.

In the case of diastereomeric mixtures (racemate mixtures) these may be separated into stereoisomeric (diastereomeric) pure racemates by means of chromatography or fractional crystallization.

Starting materials

Starting materials of the formula II

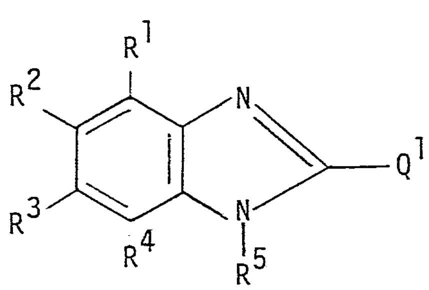

II

wherein $Q^1$ is SH may be obtained from the corresponding o-phenylenediamine by reaction with potassium ethylxanthate (Org. Synth. Vol. 30, p. 56) or thiophosgene.

The compounds of the formula II wherein $Q^1$ is alkylmercapto and alkylsulfinyl may be obtained from the above mentioned compound by simple S-alkylation with alkyl halide and by oxidation of the product from the S-alkylation, respectively.

The compounds of the formula II wherein $Q^1$ is halogen or acyloxy may be obtained from compounds of the

same formula wherein $Q^1$ is OH by treatment with $POCl_3$, $POBr_3$ and the like or the appropriate acyl halide, respectively. The starting material wherein $Q^1$ is OH is obtained from the corresponding o-phenylenediamine by reaction with phosgene.

The o-phenylenediamines required may be obtained from the corresponding substituted benzenes according to processes known per se, e.g. by the consecutive processes: nitration, reduction, acetylation, nitration, deacetylation and reduction or from one of the intermediary stages just mentioned. In order to obtain a o-phenylenediamine wherein $R^5$ is other than H, substitution is preferably made on the nitro-aniline stage.

Starting materials of the formula III

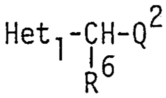 III

may be obtained from the corresponding heterocyclic compound which on the atom adjacent to the "pyridine nitrogen" is substituted with a group $P^1$ convertible to the group

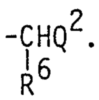

Such convertible groups $P^1$ may be

a) an alkyl group which

1) can be halogenated to the compound of formula III wherein $Q^2$ is a halogen group

2) after oxidation of the "pyridine nitrogen" to the N-oxide via a known rearrangement to the intermediate hydroxyalkyl compound and then treatment of the hydroxyalkyl compound with a halogenating agent such as thionyl chloride or O-acylating agents such as p-toluenesulfonyl chloride gives compounds of the formula III wherein $Q^2$ is halogen and sulfonyloxy groups, respectively.

b) a 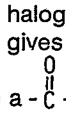 group (wherein $R^{13}$ is hydrogen, an alkyl or an alkoxy group) which after reduction to the intermediate hydroxyalkyl group and then treatment of the hydroxyalkyl group with a halogenating agent such as thionyl chloride or an O-acylating agent such as p-toluenesulfonyl chloride gives compounds of the formula III wherein $Q^2$ is halogen and sulfonyloxy groups, respectively.

The 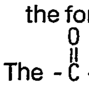 groups are reduced to hydroxyalkyl groups by treatment with a complex metal hydride e.g. lithium aluminium hydride.

The leaving groups $Q^2$ may be substituted for alkylmercapto groups by treatment with e.g. sodium alkylmercaptide, which may then be oxidized to an alkylsulfinyl group or substituted for SH by treatment with e.g. NaSH.

The intermediates of formula

$Het_1$-$P^1$

are either known in the literature or may be obtained by processes generally known for the preparation of compounds having a similar structure.

Starting materials of the formula IV

$Het_2$-$Q^2$ IV

wherein $Q^2$ is SH may be obtained from the corresponding sulfonyl chloride by reduction with lithium aluminium hydride. The sulfonyl chlorides may be obtained by reaction of the heterocyclic compound with chlorosulfuric acid.

Starting materials of the formula IV wherein $Q^2$ is halogen may be obtained from compounds of the same formula wherein $Q^2$ is OH by treatment with $POCl_3$, $POBr_3$ and the like.

The invention is illustrated by the following examples.

Example 1. Method a. Preparation of 4,6-dimethyl-2-[[(imidazo[1,2-a] pyridine-2-yl)methyl]sulfinyl]-1H-benzimidazole.

m-Chloroperbenzoic acid, 80.0% (0.77 g, 0.0036 mol) dissolved in $CH_2Cl_2$ (15 ml) was added under stirring to a cooled (0°C) mixture of 4,6-dimethyl-2-[[(imidazo[1,2-a]pyridine-2-yl)methyl]thio]-1H-benzimidazole (1.1 g, 0.0036 mol) dissolved in $CH_2Cl_2$ (50 ml) and $NaHCO_3$ (0.6 g) dissolved in water (20 ml). Stirring was continued at 0°C for 10 min and then the two phases were separated. The $CH_2Cl_2$-phase was dried ($MgSO_4$) and the solvent was evaporated off. The residue was crystallized from $CH_3CN$ yielding the desired compound (0.40 g, 34%). The identity of the product was confirmed with NMR.

Example 2. Method b. Preparation of 4,6-dimethyl-2-[[(imidazo[1,2-a]-2-pyridine-2-yl)methyl]thio]-1H-benzimidazole.

To 4,6-dimethyl-2-mercapto-1H-benzimidazole (1.8 g, 0.010 mol) in methanol (25 ml) were added (in the following order) NaOH (0.80 g, 0.020 mol) dissolved in water (10 ml) and 2-chloromethyl imidazo [1,2-a]pyridine

hydrochloride (2.0 g, 0.010 mol). The mixture was refluxed overnight and then poured on ice-water. The precipitate was filtered off and washed with water and then dissolved in $CH_2Cl_2$ (30 ml). To this solution was added under stirring 1M NaOH (30 ml). The phases were separated. The $CH_2Cl_2$-phase was dried ($MgSO_4$) and the solvent was evaporated off giving the desired compound (2.6 g, 84%). The identity of the product was confirmed with NMR.

Example 3. Method b. Preparation of 2-[4,7-dimethyl-8-quinolinyl]thio-1H-benzimidazole.

To 4,7-dimethyl-8-mercaptoquinoline (0.30 g, 0.0016 mol) in ethanol (25 ml) were added conc. HCl (0.28 ml) and 2-chlorobenzimidazole (0.25 g, 0.0016 mol). The mixture was refluxed overnight. pH was adjusted by 13.0 by an addition of 2M NaOH. Part of the solvent was evaporated off. The mixture was poured on ice-water. The precipitate was filtered off giving the desired compound (0.30 g, 62%). The identity of the product was confirmed with NMR.

Example 4. Method c. Preparation of 1-methyl-2-[8-quinolinyl]sulfinyl-1H-benzimidazole.

To NaOH (0.22 g, 0.0054 mol) dissolved in $H_2O$ (15 ml) were added under stirring (in the following order) 2-[8-quinolinyl]sulfinyl-1H-benzimidazole (0.80 g, 0.0027 mol), tetrabutylammoniumhydrogen sulfate (0.93 g, 0.0027 mol) and methyl iodide (0.20 ml, 0.033 mol) in $CH_2Cl_2$ (20 ml). The mixture was refluxed under stirring for 2 hours. The phases were separated. The organic phase was dried ($NaSO_4$), filtered and the solvent was evaporated off. Chromatography of the residue on $SiO_2$ with methylene chloride-methanol containing 1 per cent triethylamine as eluent, gave the desired compound (0.40 g, 48%). The identity of the product was confirmed with NMR.

The working examples are summarized in Table 2. Identifying data for the compounds are given in Table 3.

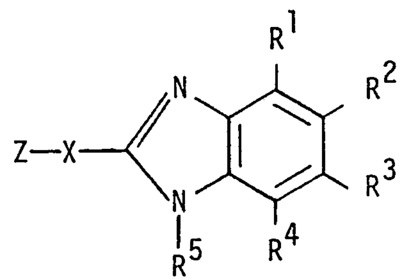

Table 2.

Summary of working examples

| Ex. | Z | X | $R^5$ | $R^1$ | $R^2$ | $R^3$ | $R^4$ | Method | Yield % | Identifying data |
|---|---|---|---|---|---|---|---|---|---|---|
| 2 | 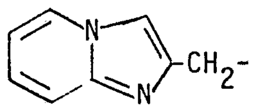 $-CH_2-$ | S | H | $CH_3$ | H | $CH_3$ | H | b | 84 | NMR |
| 1 | 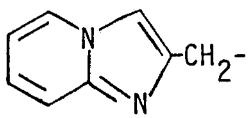 $-CH_2-$ | SO | H | $CH_3$ | H | $CH_3$ | H | a | 34 | NMR |
| 5 | 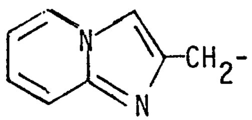 $-CH_2-$ | S | H | H | H | H | H | b | 82 | NMR |
| 6 | 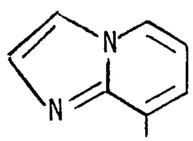 | S | H | H | H | H | H | b | | |
| 7 | 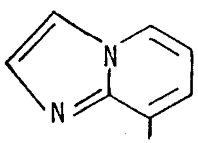 | SO | H | H | H | H | H | a | | |

cont.

| Ex. | Z | X | $R^5$ | $R^1$ | $R^2$ | $R^3$ | $R^4$ | Method | Yield % | Identifying data |
|---|---|---|---|---|---|---|---|---|---|---|
| 8 | 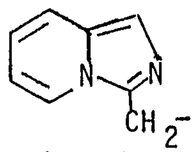 | S | H | H | $OCH_3$ | H | H | b | | |
| 9 | 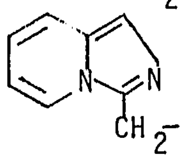 | SO | H | H | $OCH_3$ | H | H | a | | |
| 10 | 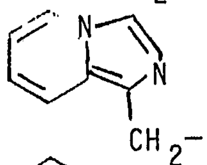 | S | H | H | $OCH_3$ | H | H | b | | |
| 11 | 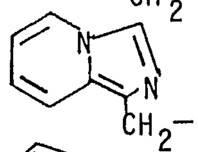 | SO | H | H | $OCH_3$ | H | H | a | | |
| 12 | 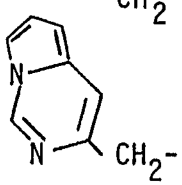 | S | H | H | $OCH_3$ | H | H | b | | |
| 13 | 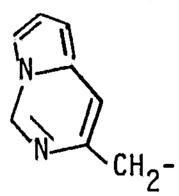 | SO | H | H | $OCH_3$ | H | H | a | | |

cont.

| Ex. | Z | X | $R^5$ | $R^1$ | $R^2$ | $R^3$ | $R^4$ | Method | Yield % | Identifying data |
|---|---|---|---|---|---|---|---|---|---|---|
| 4 | 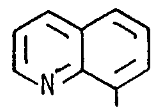 | SO | $CH_3$ | H | H | H | H | c | 48 | NMR |
| 3 | 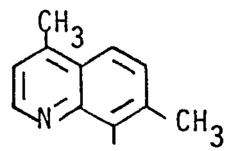 | S | H | H | H | H | H | b | 62 | NMR |
| 14 | 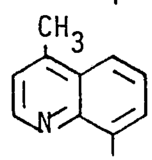 | S | $CH_3$ | H | H | H | H | b | | NMR |
| 15 | 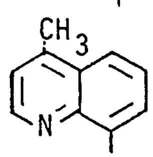 | SO | $CH_3$ | H | H | H | H | a | | |
| 16 | 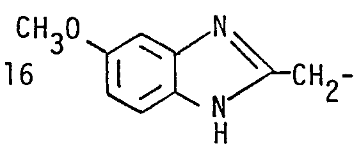 | S | H | H | $OCH_3$ | H | H | b | | NMR |
| 17 | 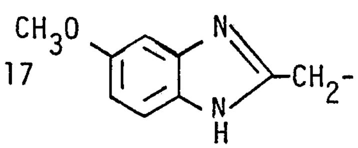 | SO | H | H | $OCH_3$ | H | H | a | 74 | NMR |

Table 3.

Identifying data for compounds of the invention.

| Ex. | Solvent | NMR data δ ppm (90 MHz) |
|---|---|---|
| 1 | $CDCl_3$ | 2.45 (s, 3H), 2.60 (s, 3H), 4.75 (dd, 2H), 6.65-7.70 (m, 6H), 8.00 (dd, 1H) |
| 2 | $CDCl_3$ | 2.45 (s, 3H), 2.65 (s, 3H), 4.40 (s, 2H), 6.75-7.75 (m, 6H), 8.15 (dd, 1H) |
| 3 | DMSO-$d_6$ (500 MHz) | 2.65 (s, 3H), 2.75 (s, 3H), 7.05-7.10 (m, 2H), 7.30-7.35 (m, 2H), 7.40 (d, 1H), 7.70 (d, 1H), 8.20 (d, 1H), 8.70 (d, 1H) |
| 4 | $CDCl_3$ | 4.25 (s, 3H), 7.20-8.05 (m, 7H), 8.20 (dd, 1H), 8.70-8.90 (m, 2H) |
| 5 | $CDCl_3$ + DMSO-$d_6$. | 4.70 (s, 2H), 6.80-7.70 (m, 7H), 7.90 (s, 1H), 8.40 (dd, 1H) |
| 14 | $CDCl_3$ | 2.65 (s, 3H), 3.85 (s, 3H), 7.10-8.00 (m, 8H), 8.90 (d, 1H) |
| 16 | $CDCl_3$ | 3.85 (s, 3H), 3.90 (s, 3H), 4.65 (s, 2H), 7.00 (dd, 2H), 7.15 (d, 2H), 7.60 (d, 2H) |
| 17 | $CDCl_3$ + DMSO-$d_6$ | 3.80 (s, 3H), 3.85 (s, 3H), 4.95 (d, 2H), 6.80-7.75 (m, 6H) |

Preparation of intermediates

Preparation of 1-hydroxymethylimidazo[1,5-a]pyridine

To a stirred solution of 1-formylimidazo[1,5-a]pyridine (600 mg, 0.0041 mol) in methanol (20 ml) was added $NaBH_4$ (150 mg, 0.0041 mol) and the solution was stirred 30 minutes at room temperature. The methanol was evaporated off and the residue was dissolved in a small amount of water. Methylene chloride was added and under stirring pH was adjusted, first to Ph 4 with concentrated HCl and after that directly to Ph 10 with NaOH (2M). The phases were separated and the water phase was extracted 3 times with methylene chloride. The combined organic layers were dried over sodium sulfate and evaporated to dryness, giving the desired compound (500 mg, 80%).

The identity of the product was confirmed with NMR;

δ(500 MHz; $CDCl_3$) 3.80 (s, 1H), 4.95 (s,2H), 6.55 (dd, 1H) 6.70 (dd, 1H), 7.55 (d, 1H), 7.85 (d, 1H), 8.05 (s, 1H).

Preparation of 2-hydroxymethyl-imidazo[1,2-a]pyridine

$LiAlH_4$ (1.4 g; 0.037 mol) was added in portions under stirring to a cooled (0°C) mixture of imidazo [1,2-a]pyridine-2-carboxylic acid ethyl ester (9.0 g, 0.047 mol) dissolved in THF (140 ml). Stirring was continued for 1 hour. To the mixture were added under stirring (in the following order) $H_2O$ (1.4 ml) dropwise, 15% NaOH (1.4 ml) and $H_2O$ (4.2 ml). The precipitated salts were filtered off and washed with THF. The THF-solution was evaporated, giving the desired compound (6.0 g, 86%).

The identity of the product was confirmed with NMR;

δ(90 MHz; $CDCl_3$) 4.90 (s, 2H), 6.65-6.90 (m, 1H), 7.05-7.30 (m, 1H), 7.50-7.70 (m, 2H), 8.10 (dd, 1H).

Preparation of 2-chloromethyl-imidazo[1,2-a]pyridine hydrochloride

Thionyl chloride (5 ml) dissolved in $CHCl_3$ (5 ml) was added dropwise under stirring to a cooled (0°C) mixture of 2-hydroxymethyl-imidazo [1,2-a] pyridine 6.0 g (0.036 mol) dissolved in $CHCl_3$ (50 ml). After stirring at room temperature for 1 hour methanol (5 ml) was added. Stirring was continued for 15 minutes. The solvent was evaporated off giving the desired compound in a quantitative yield (7.3 g).

The identity of the product was confirmed with NMR;

δ(90 MHz; $CDCl_3$ + DMSO-$d_6$) 5.00 (s, 2H), 7.40-8.20 (m, 3H), 8.70 (s,1H), 9.15 (dd, 1H).

For clinical use the compounds of the invention are formulated into pharmaceutical formulations for oral, rectal, parenteral or other mode of administration. The pharmaceutical formulation contains a compound of the invention in combination with a pharmaceutically acceptable carrier. The carrier may be in the form of a solid, semi-solid or liquid diluent, or a capsule. These pharmaceutical preparations are a further object of the invention. Usually the amount of active compounds is between 0.1-99% by weight of the preparation, between 0.2-20% by weight in preparations for parenteral use and between 1 and 50% by weight in preparations for oral administration.

In the preparation of pharmaceutical formulations containing a compound of the present invention in the form of dosage units for oral administration the compound selected may be mixed with a solid, powdered carrier, such as lactose, saccharose, sorbitol, mannitol, starch, amylopectin, cellulose derivatives, gelatin, or another suitable carrier, as well as with lubricating agents such as magnesium stearate, calcium stearate, sodium steryl fumarate and polyethylene glycol waxes. The mixture is then processed into granules or pressed into tablets. Since the sulfoxides of the invention are susceptible to degradation in acid to neutral media, granules and tablets containing sulfoxides are preferably coated with an enteric coating which protects the active compound from acid degradation as long as the dosage form remains in the stomach. The enteric coating is chosen among pharmaceutically acceptable enteric-coating materials e.g. beeswax, shellac or anionic film-forming polymers such as cellulose acetate phthalate, hydroxypropylmethyl-cellulose phthalate, partly methyl esterified methacrylic acid polymers and the like, if preferred in combination with a suitable plasticizer. To this coating various dyes may be added in order to distinguish among tablets or granules with different active compounds or with different amounts of the active compound present.

Soft gelatine capsules may be prepared with capsules containing a mixture of the active compound or compounds of the invention, vegetable oil, fat, or other suitable vehicle for soft gelating capsules. Soft gelatine capsules may also be enteric coated as described above. Hard gelatine capsules may contain granules or enteric-coated granules of the acitve compound. Hard gelatine capsules may also contain the active compound in combination with a solid powdered carrier such as lactose, saccharose, sorbitol, mannitol, potato starch, corn starch, amylopectin, cellulose derivatives or gelatine. The hard gelatine capsules may be enteric coated as described above.

Dosage units for rectal administration may be prepared in the form of suppositories which contain the active substance mixed with a neutral fat base, or they may be prepared in the form of a gelatine rectal capsule which contains the active substance in a mixture with a vegetable oil, paraffin oil or other suitable vehicle for gelatine rectal capsules, or they may be prepared in the form of a ready-made micro enema, or they may be prepared in the form of a dry micro enema formulation to be reconstituted in a suitable solvent just prior to administration.

Liquid preparations for oral administration may be prepared in the form of syrups or suspensions, e.g. solutions or suspensions containing from 0.2% to 20% by weight of the active ingredient and the remainder consisting of sugar or sugaralcohols and a mixture of ethanol, water, glycerol, propylene glycol and polyethylene glycol. If desired, such liquid preparations may contain colouring agents, flavouring agents,

saccharine and carboxymethyl cellulose or other thickening agent. Liquid preparations for oral administration may also be prepared in the form of a dry powder to be reconstituted with a suitable solvent prior to use.

Solutions for parenteral administration may be prepared as a solution of a compound of the invention in a pharmaceutically acceptable solvent, preferably in a concentration from 0.1% to 10% by weight. These solutions may also contain stabilizing agents and/or buffering agents and may be manufactured in different unit dose ampoules or vials. Solutions for parenteral administration may also be prepared as a dry preparation to be reconstituted with a suitable solvent extemporaneously before use.

The typical daily dose of the active substance varies within a wide range and will depend on various factors such as for example the individual requirement of each patient, the route of administration and the disease. In general, oral and parenteral dosages will be in the range of 5 to 500 mg per day of active substance.

Pharmaceutical preparations containing a compound of the invention as actvie ingredient are illustrated in the following examples.

Example 18. Syrup

A syrup containing 1% (weight per volume) of active substance was prepared from the following ingredients:

Active substance 1.0 g
Sugar, powder 30.0 g
Saccharine 0.6 g
Glycerol 5.0 g
Flavouring agent 0.05g
Ethanol 96% 5.0 g
Distilled water q.s. to a final volume of 100 ml

Sugar and saccharine were dissolved in 60 g of warm water. After cooling the acid addition salt was dissolved in the sugar solution and glycerol and a solution of flavouring agents dissolved in ethanol were added. The mixture was diluted with water to a final volume of 100 ml.

The above given active substance may be replaced with other pharmaceutically acceptable acid addition salts.

Example 19. Enteric-coated tablets

An enteric-coated tablet containing 20 mg of active compound was prepared from the following ingredients:

I Active substance 200 g
Lactose 700 g
Methyl cellulose 6 g
Polyvinylpyrrolidone cross-linked 50 g
Magnesium stearate 15 g
Sodium carbonate 6 g
Distilled water q.s.
II Cellulose acetate phthalate 200 g
Cetyl alcohol 15 g
Isopropanol 2000 g
Methylene chloride 2000 g

I The active compound in the form of a powder was mixed with lactose and granulated with a water solution of methyl cellulose and sodium carbonate. The wet mass was forced through a sieve and the granulate dried in an oven. After drying the granulate was mixed with polyvinylpyrrolidone and magnesium stearate. The dry mixture was pressed into tablet cores (10 000 tablets), each tablet containing 20 mg of active substance, in a tabletting machine using 6 mm diameter punches.

II A solution of cellulose acetate phthalate and cetyl alcohol in isopropanol/methylene chloride was sprayed onto the tablets I in an Accela Cota®, Manesty coating equipment. A final tablet weight of 110 mg was obtained.

Example 20. Solution for intravenous administration

A parenteral formulation for intravenous use, containing 4 mg of active compound per ml, was prepared from the following ingredients:

Active substance 4 g
Polyethylene glycol 400 for injection 400 g
Disodium hydrogen phosphate q.s.
Sterile water to a final volume of 1000 ml

The active substance was dissolved in polyethylene glycol 400 and 550 ml of water was added. pH of the solution was brought to pH 7.4 by adding a water solution of disodium hydrogen phosphate and water was added to a final volume of 1000 ml. The solution was filtered through a 0.22 μm filter and immediately dispensed into 10 ml sterile ampoules. The ampoules were sealed.

**Claims**

1. A compound of the formula

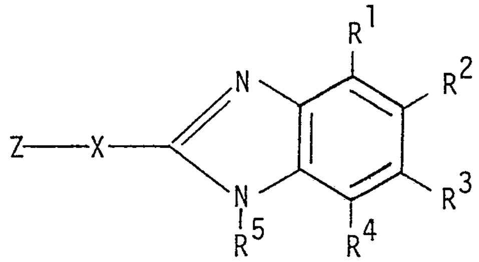

(I)

and therapeutically acceptable salts thereof, in which formula

Z is $Het_1-\underset{R_6}{\underset{|}{C}}H-$ or $Het_2-$;

X is -S- or -SO-;
$Het_1$ is a mono- or bicyclic and $Het_2$ a bicyclic aromatic heterocyclic structure containing 5-10 atoms, optionally substituted by 0-5 substituents, which are the same or different and selected from
a) alkyl containing 1-4 carbon atoms,
b) alkoxy containing 1-3 carbon atoms,
c) amino, mono- or dialkylamino containing 1-3 carbon atoms in the alkyl part(s),
d) alkylthio, containing 1-3 carbon atoms,
e) aryl,
f) arylalkyl containing 1-2 carbon atoms in the alkyl part,
g) aryloxy,
h) arylthio,
i) arylalkoxy containing 1-2 carbon atoms in the alkoxy part or
j) halogen;
Said heterocyclic structure always containing a nitrogen atom which is separated from the -X- group in formula I by two atoms, of which one, namely that one connected to the -X- group, is a carbon atom;
$R^6$ is H, $CH_3$ or $C_2H_5$;
$R^1$, $R^2$, $R^3$ and $R^4$, which are the same or different, are
a) H,
b) alkyl containing 1-6 carbon atoms,
c) cycloalkyl containing 3-7 carbon atoms,
d) alkoxy containing 1-6 carbon atoms,
e) alkoxyalkyl containing 1-3 carbon atoms in each alkyl part,
f) alkoxyalkoxy containing 1-3 carbon atoms in each alkyl part,
g) halogen,
h) -CN,
i) $-CF_3$,
j) $-NO_2$
k) $COR^7$,
l) alkylthio containing 1-6 carbon atoms in the alkyl part,
m) alkylsulfinyl containing 1-7 carbon atoms in the alkyl part,
n) aryl,
o) arylalkyl containing 1-6 carbon atoms in the alkyl part,
p) aryloxy,
q) arylalkoxy containing 1-6 carbon atoms in the alkyl part or
r) $R^1$ and $R^2$, $R^2$ and $R^3$ or $R^3$ and $R^4$ together with the adjacent carbon atoms in the benzimidazole ring form one or more 5-, 6- or 7-membered rings, which each may be saturated or unsaturated and may contain 0-3 hetero atoms selected from N, S and O and whereby each ring may be optionally substituted with 1-10 substituents selected from alkyl groups with 1-3 carbon atoms, or two or four of the mentioned substituents together form one or two oxo groups ($-\overset{O}{\overset{\|}{C}}-$), whereby if $R^1$ and $R^2$, $R^2$ and $R^3$ or $R^3$ and $R^4$ together with

the adjacent carbon atoms in the benzimidazole ring form two rings, the rings may be condensed with each other;

$R^5$ is

a) H,

b) alkyl containing 1-6 carbon atoms,

c) alkoxyalkyl containing 1-3 carbon atoms in each alkyl part,

d) aryl,

e) arylalkyl containing 1-4 carbon atoms in the alkyl part,

f) aryloxyalkyl containing 1-4 carbon atoms in the alkyl part,

g) alkylthioalkyl containing 1-3 carbon atoms in the alkyl parts,

h) carboxy-substituted alkyl containing 1-6 carbon atoms, optionally in the form of a salt such as the sodium or potassium salt,

i) amino-, monoalkyl (1-3 carbon atoms) -amino-, and dialkyl (1-3 carbon atoms in each alkyl part) -amino-substituted alkyl containing 1-6 carbon atoms, optionally in the form of a salt such as the hydrochloride,

j) cyanoalkyl containing 1-4 carbon atoms in the alkyl part,

k) arylthioalkyl containing 1-4 carbon atoms in the alkyl part,

l) 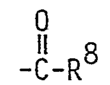 or

m) 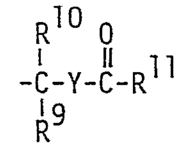

Y is -O- or $-NR^{12}-$;

$R^7$ is

a) alkyl containing 1-6 carbon atoms or

b) alkoxy containing 1-6 carbon atoms;

$R^8$ is

a) alkyl containing 1-6 carbon atoms,

b) arylalkyl containing 1-2 carbon atoms in the alkyl part,

c) aryl,

d) alkoxy containing 1-4 carbon atoms,

e) arylalkoxy containing 1-2 carbon atoms in the alkyl part,

f) aryloxy,

g) amino,

h) mono- or dialkylamino containing 1-4 carbon atoms in the alkyl part(s),

i) arylalkylamino containing 1-2 carbon atoms in the alkyl part or

j) arylamino;

$R^9$ and $R^{10}$ are the same or different and selected from

a) H or

b) alkyl containing 1-4 carbon atoms;

$R^{11}$ is

a) alkyl containing 1-6 carbon atoms,

b) mono- or dihydroxy-substituted alkyl containing 1-6 carbon atoms,

c) amino-, monoalkyl (1-3 carbon atoms) -amino-, and dialkyl (1-3 carbon atoms in each alkyl part) -amino-substituted alkyl containing 1-6 carbon atoms, optionally in the form of a salt such as the hydrochloride,

d) carboxy-substituted alkyl containing 1-7 carbon atoms, optionally in the form of a salt such as the sodium or potassium salt,

e) cycloalkyl containing 3-7 carbon atoms,

f) alkoxy containing 1-6 carbon atoms,

g) mono- or dihydroxysubstituted alkoxy containing 1-6 carbon atoms,

h) amino-, monoalkyl (1-3 carbon atoms) -amino-, and dialkyl (1-3 carbon atoms in each alkyl part) -amino-substituted alkoxy containing 1-6 carbon atoms, optionally in the form of a salt such as the hydrochloride,

i) carboxy-substituted alkoxy containing 1-7 carbon atoms, optionally in the form of a salt such as the sodium or potassium salt,

j) alkoxyalkoxy containing 1-3 carbon atoms in each alkyl part,

k) alkylamino containing 1-6 carbon atoms,

l) dialkylamino containing 1-4 carbon atoms in each alkyl part,

m) aryl, aryloxy or arylalkoxy containing 1-6 carbon atoms in the alkoxy part, the aryl groups are optionally substituted with alkyl containing 1-4 carbon atoms, alkoxy containing 1-4 carbon atoms, halogen, $CF_3$,

alkanoyl containing 2-5 carbon atoms, alkoxycarbonyl containing 2-5 carbon atoms or carboxy, whereby the carboxy group optionally may be in the form of a salt such as the sodium salt;

$R^{12}$ is

a) H or

b) alkyl containing 1-4 carbon atoms.

With the provisos that

1) $Het_1$ is neither a pyridine ring nor a pyridine ring condensed with a carbocyclic ring or with an O- or N- containing ring including only one heteroatom, which is attached to the 4-position of the pyridine ring

2) $Het_1$ is imidazolyl or thiazolyl, unsubstituted or monosubstituted by alkyl or halogen only when a) at least three of $R^1$, $R^2$, $R^3$ and $R^4$ are other than hydrogen, b) $R^1$, $R^2$, $R^3$ and $R^4$ form at least one ring structure, or c) when $R^5$ is the group $CR^9R^{10}YCOR^{11}$

3) when $Het_1$ is benzimidazolyl substituted by up to two substituents in the benzene part and unsubstituted or substituted by alkyl, acyl or alkoxycarbonyl on one of the nitrogen atoms, then $R^1$, $R^2$, $R^3$ and $R^4$ are not all hydrogen or when three of $R^1$, $R^2$, $R^3$ and $R^4$ are hydrogen, the fourth is not alkyl or halogen.

4) when $R^5$ is hydrogen and $Het_2$ is a quinoline ring, this ring is substituted in at least one of the positions 5, 6 and 7.

2. A compound according to claim 1 wherein the radical Z contains a $sp^2$-hybridized nitrogen atom, separated from the radical X by two carbon atoms.

3. A compound according to claim 2 wherein the radical Z contains an unsubstituted carbon atom next to the $sp^2$-hybridized nitrogen atom.

4. A compound according to claim 1-3 wherein the radical Z a) contains a nitrogen atom separated from the radical X by two carbon atoms and b) has a pKa-value of 2 or more.

5. A compound according to claim 1 wherein the radical Z is selected from the group consisting of

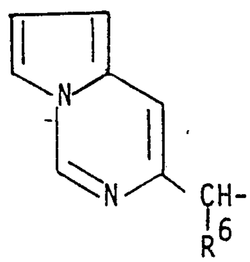
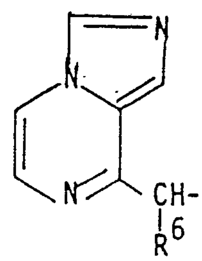
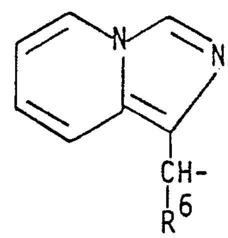
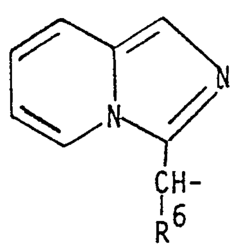
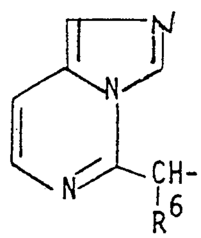
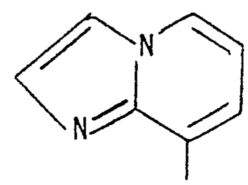
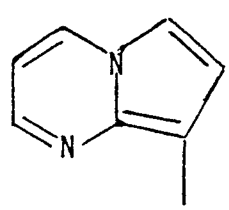
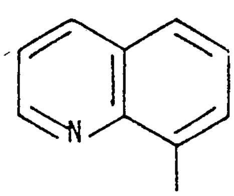
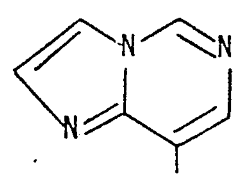

which may be unsubstituted or substituted by 1-3 substituents as specified in claim 1.

6. A pharmaceutical composition containing as active ingredient a compound according to any of claims 1-5.

7. A compound as defined in any of claims 1-5, or a therapeutically acceptable salt thereof, for use in therapy.

8. A compound as defined in any of claims 1-5, or a therapeutically acceptable salt thereof, for use in

inhibiting gastric acid secretion in mammals and man.

9. A compound as defined in any of claims 1-5, or a therapeutically acceptable salt thereof, for use as a gastrointestinal cytoprotecting agent in mammals and man.

10. A compound as defined in any of claims 1-5, or a therapeutically acceptable salt thereof, for use in the treatment of gastrointestinal inflammatory diseases in mammals and man.

11. A process for the preparation of a compound according to claim I by

a) Oxidizing a compound of the formula I

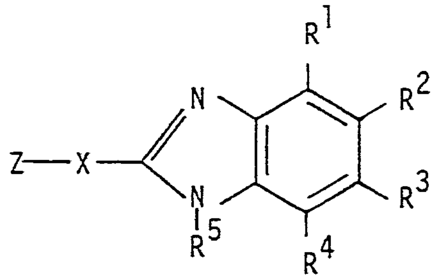

I

wherein X is S and Z, $R^1$, $R^2$, $R^3$, $R^4$ and $R^5$ have the meanings in claim 1 to give a compound of formula I wherein X is SO;

b) Reacting a compound of the formula II

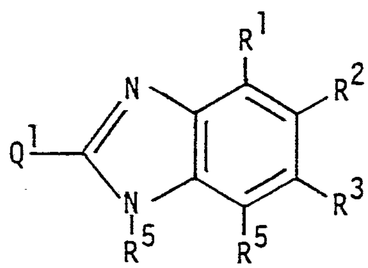

II

with a compound of the formula III or IV

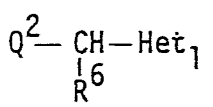

III

$Q^2$—$Het_2$ IV

in which $R^1$, $R^2$, $R^3$, $R^4$, $R^5$, $R^6$, $Het_1$ and $Het_2$ are as defined in claim 1 and wherein one of $Q^1$ and $Q^2$ is SH and the other is a leaving group, to give a compound of the formula I wherein X is S;

c) Reacting a compound of the formula V c) Reacting a compound of the formula V

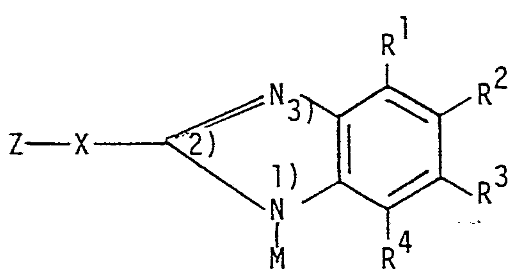

V

wherein $R^1$, $R^2$, $R^3$, $R^4$, X and Z are as defined in claim 1 and M is either a metal cation such as Na+, K+ and Li+ or a quaternary ammonium ion, such as tetrabutylammonium, with a compound of the formula VI

$R^5Q^3$ VI

wherein $R^5$ is as defined under formula I and $Q^3$ is halogen such as Cl, Br or I, or a functionally equivalent group;

whereafter the compound of the formula I obtained if desired, when X is -S-, is converted to a physiologically acceptable salt or oxidized to form a compound of the formula I wherein X is -SO-.

European Patent Office

# EUROPEAN SEARCH REPORT

Application number
EP 87850049.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | DE-A1-25 04 252 (HÄSSLE) | 1-11 | C 07 D 401/12, 403/12, 471/04, 487/04; A 61 K 31/415, 31/44 |
| X | DE-A1-25 48 340 (HÄSSLE) | 1-11 | |
| X | EP-A1-0 001 279 (HOFFMANN-LA ROCHE) | 1-11 | |
| X | EP-A1-0 045 200 (THE UPJOHN) | 1-10 | |
| | | | **TECHNICAL FIELDS SEARCHED (Int. Cl.4)** |
| | | | C 07 D<br>A 61 K |

The present search report has been drawn up for all claims

| Place of search | Date of completion of the search | Examiner |
|---|---|---|
| STOCKHOLM | 11-05-1987 | CARLQVIST B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82